# EUROPEAN PATENT APPLICATION

(11) **EP 4 389 821 A1**
(43) Date of publication of application: **26.06.2024**
(21) Application number: 22858499.1
(22) Date of filing: 17.08.2022
(51) Int. Cl.: C08L 29/04, C08G 81/02, C08L 89/00, C12N 11/084

(54) **HYDROGEL AND STERILIZED DRY HYDROGEL-FORMING ARTICLE**

(30) Priority: 18.08.2021 JP 2021133378; 18.08.2021 JP 2021133384
(71) Applicant: Kuraray Co., Ltd., Kurashiki-shi, Okayama 710-0801 (JP)
(72) Inventor: FUJITA, Akio, Tsukuba-shi, Ibaraki 305-0841 (JP); AYANO, Satoru, Tsukuba-shi, Ibaraki 305-0841 (JP); KOBAYASHI, Goro, Tsukuba-shi, Ibaraki 305-0841 (JP)
(74) Representative: Müller-Boré & Partner Patentanwälte PartG mbB
(86) International application number: PCT/JP2022/031127
(87) International publication number: WO 2023/022181

(57) **Abstract**

A sterilized dry hydrogel-forming article comprising a crosslinked product of a vinyl alcohol polymer having a bioactive substance conjugated thereto. A hydrogel comprising a crosslinked product of a vinyl alcohol polymer having an ethylenically unsaturated group and a carboxy group and a bioactive substance having an amino group, the carboxy group of the crosslinked product and the amino group of the bioactive substance being covalently bound via an amide bond.

## Description

### Technical Field

### Cross-reference to Related Patent Applications

The present application claims priority to Japanese Patent Application No. 2021-133378 and Japanese Patent Application No. 2021-133384, filed on August 18, 2021, the entire disclosure of which is hereby incorporated by reference. The present invention relates to a hydrogel and a sterilized dry hydrogel-forming article.

### Background Art

Polyvinyl alcohol (abbreviated as "PVA" below) is a water-soluble synthetic polymer with excellent features, such as hydrophilicity, reactivity, biodegradability, biocompatibility, and low toxicity, and can be physically or chemically crosslinked to form a hydrogel with high levels of flexibility and strength. Furthermore, the hydrogel conjugated with a bioactive substance ("conjugated hydrogel" below) exhibits specific functionality; thus, applications of the hydrogel are suggested, for example, in enzyme immobilization carriers (e.g., NPL 1), affinity carriers (e.g., NPL 2), cell culture substrates (e.g., NPL 3), particles for vascular embolization (e.g., PTL 1), and cell culture carriers (e.g., PTL 2).

The conjugated hydrogel must be used in a sterilized condition, in particular, in pharmaceutical products or in the manufacture of parts and components of medical equipment used in the human body, among these applications. An unsterilized conjugated hydrogel used in these applications would cause infection due to residual microorganisms and result in fatal damage to organisms or cells. Thus, it is desirable for the conjugated hydrogel to achieve an SAL of 10⁻⁶ or less as the sterility assurance level ("SAL" below) for these applications.

Sterilizing or drying a conjugated hydrogel is known to be difficult because bioactive substances that contain enzymes generally lose their original activity under harsh sterilization or drying conditions. For example, it has been known that a hydrogel conjugated with a cell adhesion protein (bioactive substance) can be suitably used as a cell culture substrate. In these prior art techniques, a prepared conjugated hydrogel is irradiated with UV light for several hours or immersed in a 70 to 75% aqueous ethanol solution for several hours before being used for cell culture (e.g., NPL 3 to 5). Although UV light irradiation or immersion in an aqueous ethanol solution is unlikely to cause enough damage for the bioactive substance to lose activity, these treatments cannot sterilize the conjugated hydrogel to such a level as to achieve an SAL of 10⁻⁶ or less.

Attempts also have been made to produce a sterile conjugated hydrogel (e.g., NPL 6). A study used a conjugated PVA in a sol form prepared by introducing an acrylamide group as a crosslinking site and an RGDS peptide as a cell adhesion protein into a low-molecular-weight PVA. This study discloses that a sterile conjugated hydrogel can be produced by subjecting the aqueous solution to filtration sterilization by using a filter and aseptically curing it.

Another method commonly used in chemically crosslinking a hydrogel is using glutaraldehyde as a crosslinking agent. There are methods already disclosed, such as a method of covalently bonding a hydrogel chemically crosslinked by glutaraldehyde with an enzyme by using carbonyldiimidazole (e.g., NPL 1), and a method of covalently bonding a cell adhesion protein with PVA having a carboxy group introduced (e.g., NPL 3 and 4). However, glutaraldehyde (crosslinking agent) is highly toxic, and it is undeniable that a small amount of glutaraldehyde is leached from a chemically crosslinked hydrogel. Although such a hydrogel can be used for research purposes, it is thus difficult to put it to practical use in the applications as described above.

To solve the problem of the chemical crosslinking of PVA by using glutaraldehyde, some attempts have been made to produce a hydrogel that is crosslinked due to radical polymerization evoked by the introduction of an acrylamide group into PVA and that has a covalently bonded cell adhesion peptide (e.g., NPL 6 and 7). Similarly, a study discloses a hydrogel prepared by crosslinking PVA having a (meth)acryloyl group by radical polymerization to prepare PVA particles and covalently bonding the PVA particles with cell-adhesive gelatin (e.g., PTL 2) .

### Citation List

### Patent Literature

PTL 1: JP2002-527206A
PTL 2: WO2020/105708A

### Non-patent Literature

NPL 1: Food Chemistry, 2001, Vol. 74, pp. 281-288
NPL 2: Biotechnology Techniques, 1997, Vol. 11, pp. 67-70
NPL 3: Journal of Biomedical Materials Research, 2001, Vol. 57, pp. 217-223
NPL 4: Journal of Polymer Engineering, 2017, Vol. 37, pp. 647-660
NPL 5: Journal of Applied Biomaterials, 1991, Vol. 2, pp. 261-267
NPL 6: Biomaterials, 2002, Vol. 23, pp. 4325-4332
NPL 7: Biomaterials, 2012, Vol. 33, pp. 3880-3886

### Summary of Invention

### Technical Problem

Although the conjugated hydrogel irradiated with UV light irradiation or immersed in an ethanol solution as described in NPL 3 to 5 can be used for short-term cell culture testing, the risk of infection due to microorganisms is not eliminated in application in cell culture processes or to the human body due to insufficient sterilization.

Furthermore, the filtration sterilization disclosed in NPL 6 requires a sterile environment for curing and packaging after sterilization. The production of sterilized conjugated hydrogels in a sterile environment is very costly. Additionally, it is essential to use low-molecular-weight PVA (average molecular weight: 13,000) in the filtration sterilization of conjugated hydrogels in a sol form (a molecular weight higher than this resulting in substantial pressure loss, making the filtration sterilization impossible); however, the resulting conjugated hydrogels become fragile and have limited applications.

In an embodiment, the present invention was made in view of the problems above, and an object of the present invention is to provide a sterilized conjugated hydrogel-forming article without using expensive processes, such as a sterile environment.

The methods disclosed in NPL 6 and 7 can solve the problem with chemical crosslinking by glutaraldehyde. However, a problem remains in the method of introducing a bioactive substance or an enzyme. More specifically, the prior techniques introduce a carboxy group derived from glutamic acid, aspartic acid residues, etc. of a bioactive substance (including enzymes) into an amino group incorporated in PVA by using a carbodiimide condensation agent. However, because the bioactive substance usually contains not only carboxy groups but also amino groups derived from lysine residues, etc., self-crosslinking also occurs at the same time. This interferes with efficient introduction into a hydrogel. Additionally, the method using carbonyldiimidazole described in PTL 2 is unable to introduce a bioactive substance or an enzyme at a density high enough to be used in the applications described above.

In an embodiment, the present invention was made in view of the problems described above, and an object of the present invention is to provide a hydrogel into which a bioactive substance or an enzyme has been efficiently introduced by covalent bonding by using a less toxic crosslinking method.

### Solution to Problem

The present inventors conducted extensive research. As a result, they found that when a conjugated hydrogel-forming article is in a dry state, which is achieved by removing water from the conjugated hydrogel-forming article, the conjugated hydrogel-forming article can be sterilized while bioactive substance activity is maintained. Further, the present inventors conducted extensive research. As a result, they found that when a vinyl alcohol polymer having an ethylenically unsaturated group and a carboxy group is used, a hydrogel can be produced by a crosslinking method with low toxicity, unlike the method of crosslinking using glutaraldehyde, and a bioactive substance having more amino groups than hydrogel crosslinked with glutaraldehyde can be efficiently introduced via a covalent bond. The present invention has been accomplished based on these findings. In the present specification, the "conjugated hydrogel" means a crosslinked product of a vinyl alcohol polymer having a bioactive substance conjugated thereto.

Specifically, the present invention relates to the following [1] to [11].
[1] A sterilized dry hydrogel-forming article comprising a crosslinked product of a vinyl alcohol polymer having a bioactive substance conjugated thereto.
[2] The dry hydrogel-forming article according to [1], wherein the bioactive substance is conjugated to the crosslinked product of the vinyl alcohol polymer via a covalent bond.
[3] The dry hydrogel-forming article according to [1] or [2], wherein the dry hydrogel-forming article has a water content of 75 mass% or less.
[4] The dry hydrogel-forming article according to any one of [1] to [3], wherein the dry hydrogel-forming article is non-spherical particles, spherical particles, a fine molded article, an article of any shape molded with a 3D printer, a film, thread, hollow fibers, a porous monolith, or a coated article.
[5] The dry hydrogel-forming article according to any one of [1] to [4], wherein the dry hydrogel-forming article has a sterility assurance level (SAL) of 10⁻³ or less.
[6] A method for producing a dry hydrogel-forming article of any one of [1] to [5], the method comprising sterilizing a hydrogel-forming article in a dry state.
[7] The method according to [6], wherein the sterilization is performed by a radiation sterilization method.
[8] The method according to [7], wherein the radiation dose is 8.2 kilo Grays (kGy) or more.
[9] The method according to [7] or [8], wherein the dry hydrogel-forming article is kept in a container and then irradiated with radiation.
[10] The dry hydrogel-forming article according to any one of [1] to [5], which is kept in a container.
[11] A hydrogel comprising
   a crosslinked product of a vinyl alcohol polymer having an ethylenically unsaturated group and a carboxy group; and
   a bioactive substance having an amino group,
   the carboxy group of the crosslinked product and the amino group of the bioactive substance being covalently bound via an amide bond.
[12] The hydrogel according to [11], wherein the ethylenically unsaturated group is at least one member selected from the group consisting of vinyl, (meth)acryloyl, (meth)acryloylamino, vinylphenyl, norbornenyl, and derivatives thereof.
[13] The hydrogel according to [11] or [12], wherein an introduction rate of the ethylenically unsaturated group is 0.01 to 10 mol%, based on all structural units constituting the vinyl alcohol polymer.
[14] The hydrogel according to any one of [11] to [13], wherein an introduction rate of the carboxy group is 0.1 to 50 mol%, based on all structural units constituting the vinyl alcohol polymer.
[15] The hydrogel according to any one of [11] to [14], wherein the hydrogel is non-spherical particles, spherical particles, a fine molded article, an article of any shape molded with a 3D printer, a film, thread, hollow fibers, a porous monolith, or a coated article.

### Advantageous Effects of Invention

In one the embodiment, the present invention can provide a sterilized conjugated hydrogel-forming article without using an expensive process, such as a sterile environment. In another embodiment, by using a hydrogel comprising a crosslinked product of a vinyl alcohol polymer having an ethylenically unsaturated group and a carboxy group, the present invention can provide a low-toxicity hydrogel having a bioactive substance or an enzyme efficiently introduced thereinto via a covalent bond, wherein the bioactive substance having an amino group is covalently bound to the carboxy group via an amide bond.

### Description of Embodiments

### 1. Dry Hydrogel-forming Article

In a first embodiment, the present invention provides a sterilized dry hydrogel-forming article comprising a crosslinked product of a vinyl alcohol polymer having a bioactive substance conjugated thereto. The hydrogel contained in the dry hydrogel-forming article of the present invention is preferably a hydrogel comprising a crosslinked product of a vinyl alcohol polymer having an ethylenically unsaturated group and a carboxy group, and is preferably a hydrogel in which ae bioactive substance having an amino group is covalently bound to a carboxy group of a vinyl alcohol polymer via an amide bond.

### Dry Hydrogel-forming Article

The dry hydrogel-forming article of the present invention is a molded article comprising a conjugated hydrogel as described above, which is in a dried state. The dry hydrogel-forming article of the present invention swells to form a hydrogel when it comes into contact with an aqueous solution, such as water, buffer, body fluid, or culture medium. The molded article is in the form of, for example, general non-spherical particles, spherical particles, a film, thread, hollow fibers, or a porous monolith.

Further, the molded article may be, for example, in the form of a fine molded article, an article of any shape molded with a 3D printer, or an article coated with a dry hydrogel-forming article (coated article). The fine molded article is a molded article having fine asperities on its surface and inside, and having a fine processing size of 10 to 1000 µm. The article of any shape molded with a 3D printer is, for example, an arbitrarily shaped object that can be created with a stereolithographic, ink jet, or nozzle extrusion 3D printer. The coated article is an article comprising a substrate and a conjugated hydrogel, the substrate being in the form of a film, a tray, thread, hollow fiber, a porous monolith, a fine molded article, or an article of any shape molded with a 3D printer and the substrate being coated with the conjugated hydrogel. The material of the substrate can be freely selected from, for example, glass, polyolefin, polymethyl methacrylate, polystyrene, polyester, polyethylene-vinyl alcohol copolymer, polyamide, and polyimide.

### Vinyl Alcohol Polymer and Production Method Therefor

The vinyl alcohol polymer used in the present invention can be produced by saponifying a polyvinyl ester obtained by polymerizing a vinyl ester monomer, and then converting the ester group in the polyvinyl ester into a hydroxyl group.

Examples of the vinyl ester monomer include aliphatic vinyl esters, such as vinyl formate, vinyl acetate, vinyl propionate, vinyl n-butyrate, vinyl isobutyrate, vinyl pivalate, vinyl versatate, vinyl caproate, vinyl caprylate, vinyl caprate, vinyl laurate, vinyl myristate, vinyl palmitate, vinyl stearate, and vinyl oleate; and aromatic vinyl esters, such as vinyl benzoate. Such vinyl ester monomers can be used alone or in a combination of two or more.

Among the vinyl ester monomers, aliphatic vinyl esters are preferred. From the viewpoint of production costs, vinyl acetate is more preferred. That is, the polyvinyl ester is preferably polyvinyl acetate obtained by polymerizing vinyl acetate.

The polyvinyl ester may contain structural units derived from monomers other than vinyl ester monomers, if necessary, as long as the effect of the present invention is not impaired. Examples of such other monomers include α-olefins, such as ethylene, propylene, n-butene, and isobutylene; acrylic acid or salts thereof; alkyl acrylates, such as methyl acrylate, ethyl acrylate, n-propyl acrylate, i-propyl acrylate, n-butyl acrylate, i-butyl acrylate, t-butyl acrylate, 2-ethylhexyl acrylate, dodecyl acrylate, and octadecyl acrylate; methacrylic acid or salts thereof; alkyl methacrylates, such as methyl methacrylate, ethyl methacrylate, n-propyl methacrylate, i-propyl methacrylate, n-butyl methacrylate, i-butyl methacrylate, t-butyl methacrylate, 2-ethylhexyl methacrylate, dodecyl methacrylate, and octadecyl methacrylate; acrylamide derivatives, such as acrylamide, N-methylacrylamide, N-ethylacrylamide, N,N-dimethylacrylamide, diacetoneacrylamide, acrylamidopropane sulfonic acid or salts thereof, acrylamidopropyl dimethylamine or salts or quaternary salts thereof, N-methylolacrylamide or derivatives thereof; methacrylamide derivatives, such as methacrylamide, N-methylmethacrylamide, N-ethylmethacrylamide, methacrylamidopropanesulfonic acid or salts thereof, methacrylamidopropyl dimethylamine or salts or quaternary salts thereof, and N-methylolmethacrylamide or derivatives thereof; N-vinylamide derivatives, such as N-vinylformamide and N-vinylacetamide; vinyl ethers, such as methyl vinyl ether, ethyl vinyl ether, n-propyl vinyl ether, i-propyl vinyl ether, n-butyl vinyl ether, i-butyl vinyl ether, t-butyl vinyl ether, dodecyl vinyl ether, and stearyl vinyl ether; nitriles, such as acrylonitrile and methacrylonitrile; vinyl halides, such as vinyl chloride and vinyl fluoride; vinylidene halides, such as vinylidene chloride and vinylidene fluoride; allyl compounds, such as allyl acetate and allyl chloride; maleic acid or salts thereof, ester or acid anhydride; vinyl silyl compounds, such as vinyl trimethoxysilane; and isopropenyl acetate. Such other monomers can be used alone or in a combination of two or more.

When the polyvinyl ester contains structural units derived from other monomers, the content of structural units derived from other monomers is preferably 20 mol% or less, more preferably 10 mol% or less, and even more preferably 5 mol% or less, based on all structural units constituting the polyvinyl ester.

The method for saponifying the polyvinyl ester is not particularly limited, and the same method as conventionally used can be used. Examples of usable methods include the alcoholysis method, the hydrolysis method, and the like using an alkali catalyst or an acid catalyst. In particular, a saponification reaction using methanol as a solvent and using a caustic soda (NaOH) catalyst is simple and preferred.

From the viewpoint of suppressing embrittlement of the crosslinked product of the vinyl alcohol polymer during water swelling, the degree of polymerization of the vinyl alcohol polymer is preferably 300 or more, more preferably 350 or more, even more preferably 400 or more, and particularly preferably 450 or more. From the viewpoint of inhibiting high viscosity of the aqueous solution and improving ease of processing when the crosslinked product of the vinyl alcohol polymer is produced, the degree of polymerization of the vinyl alcohol polymer is preferably 10000 or less, more preferably 5000 or less, and even more preferably 3500 or less. Two or more vinyl alcohol polymers of different degrees of polymerization may be mixed together and used.

The degree of polymerization of the vinyl alcohol polymer in the present specification refers to the degree of polymerization measured according to JIS K 6726:1994. Specifically, the degree of polymerization can be determined from the intrinsic viscosity that is measured in water at 30°C after the starting material PVA has been saponified and purified.

The degree of saponification of the vinyl alcohol polymer is preferably 50 mol% or more, more preferably 60 mol% or more, and even more preferably 65 mol% or more, from the viewpoint of improving the water solubility of the vinyl alcohol polymer. The upper limit of the degree of saponification is 100 mol% or 99 mol%.

In the present specification, the degree of saponification of the vinyl alcohol polymer means the ratio (mol%) of the number of moles of the vinyl alcohol unit to the total number of moles of the structural unit (e.g., vinyl acetate unit) that can be converted to a vinyl alcohol unit by saponification in the starting material PVA and the vinyl alcohol unit. The saponification degree of the starting material PVA can be measured according to JIS K 6726:1994.

The 4 mass% viscosity at 20°C of the starting material PVA is preferably 0.5 to 110 mPa·s, more preferably 1 to 80 mPa·s, and even more preferably 2 to 60 mPa·s. When the viscosity is within these ranges, the hydrogel can be more easily produced, and the strength of the conjugated hydrogel can be enhanced.

In the present specification, the viscosity refers to the viscosity of an aqueous solution containing 4 mass% of the vinyl alcohol polymer as measured at a temperature of 20°C using a B-type viscometer (rotation rate: 12 rpm) according to the rotational viscometer method of JIS K 6726:1994.

### Crosslinking Product of Vinyl Alcohol Polymer

The crosslinked product of the vinyl alcohol polymer may be physically crosslinked by a vinyl alcohol polymer or may be crosslinked via a covalent bond. The total amount of vinyl alcohol-derived structural units and vinyl ester-derived structural units relative to all structural units constituting the vinyl alcohol polymer is preferably 80 mol% or more, more preferably 90 mol% or more, and even more preferably 95 mol% or more.

The method for physically crosslinking a vinyl alcohol polymer includes, for example, a method comprising freezing and thawing an aqueous solution of a vinyl alcohol polymer, a method comprising dissolving a vinyl alcohol polymer in a mixed solvent of dimethyl sulfoxide and water and cooling the heated solution to room temperature. The method for crosslinking a vinyl alcohol polymer via a covalent bond is, for example, a method using a multifunctional crosslinking agent that reacts with a side-chain hydroxyl group of a vinyl alcohol polymer, or a method comprising introducing a functional group into a vinyl alcohol polymer by copolymerization or post-modification and allowing them to react. However, it is preferable from the viewpoint of enhanced stability of the hydrogel during water swelling that the hydrogel is crosslinked via a covalent bond.

The method using a multifunctional crosslinking agent that reacts with the side chain hydroxyl group of a vinyl alcohol polymer includes a method comprising reacting a multifunctional aldehyde compound, such as glyoxal, malondialdehyde, or glutaraldehyde, under acidic conditions (polyacetal crosslinking), a method comprising reacting a multifunctional epoxy compound, such as epichlorohydrin or ethylene glycol diglycidyl ether, under alkaline conditions (polyether crosslinking), and a method comprising reacting a multifunctional carboxylic acid compound, such as maleic acid and succinic acid (polyester crosslinking).

### Modified PVA

A method for introducing a functional group into a vinyl alcohol by copolymerization is, for example, a method comprising copolymerizing a vinyl ester monomer with a polymerizable monomer that is other than a vinyl ester monomer and that has a reactive substituent other than a hydroxyl group in the process of producing the vinyl alcohol polymer, then saponifying the resulting copolymer to give copolymerized, modified polyvinyl alcohol (which may be simply referred to below as "copolymerized, modified PVA"), and then adding a multifunctional crosslinking agent capable of reacting with a functional group, such as the carboxy group present in copolymerized, modified PVA, or the amino group present in copolymerized, modified PVA. The copolymerized, modified PVA containing a carboxy group may be referred to as "carboxylic acid-modified PVA." The copolymerized, modified PVA containing an amino group may be referred to as "amino-modified PVA."

Examples of monomers that can be used to form carboxylic acid-modified PVA include α,β-unsaturated carboxylic acids, such as (meth)acrylic acid, maleic acid, fumaric acid, and itaconic acid; alkyl (meth)acrylates, such as methyl (meth)acrylate and ethyl (meth)acrylate; α,β-unsaturated carboxylic acid anhydrides and derivatives thereof, such as maleic anhydride and itaconic anhydride; and derivatives thereof. A hydrogel can be obtained by copolymerizing a vinyl ester monomer with a monomer constituting carboxylic acid-modified PVA, then saponifying the resulting copolymer to give carboxylic acid-modified PVA, and combining, for example, a multifunctional crosslinking agent capable of reacting with the introduced carboxy group, such as a multifunctional epoxy compound, such as epichlorohydrin or ethylene glycol diglycidyl ether, a multifunctional amino compound, such as ethylenediamine, polyethylene imines, or polyallylamine, and a carbodiimide condensing agent, such as 1-(3-dimethylaminopropyl)-3-ethylcarbodiimide hydrochloride. The carboxy group introduced into the carboxylic acid-modified PVA can function to covalently bind a bioactive substance having an amino group via an amide bond (-CONH-) to form a conjugate.

For amino-modified PVA, a hydrogel can be obtained by copolymerizing, for example, a vinyl ester monomer and N-vinylform amide, then saponifying the resulting copolymer, and combining, for example, a multifunctional crosslinking agent capable of reacting with the introduced amino group, such as a multifunctional epoxy compound as described above, a multifunctional carboxylic acid compound, such as succinic acid or maleic acid, and a carbodiimide condensing agent as described above.

The method comprising introducing a functional group into a vinyl alcohol polymer by post-modification and reacting them includes, for example, a method comprising introducing an ethylenically unsaturated group into the side chain of a vinyl alcohol polymer. The introduced ethylenically unsaturated group can easily form a hydrogel by adding an additive, such as a radical initiator, to induce a polymerization reaction. The introduction of an ethylenically unsaturated group is preferably performed via a side chain, a terminal functional group, or the like of the vinyl alcohol polymer. A compound containing an ethylenically unsaturated group (which may be simply referred to below as an "ethylenically unsaturated group-containing compound") is more preferably reacted with a hydroxyl group on a side chain of a vinylalcohol polymer.

Examples of the ethylenically unsaturated group-containing compound to be reacted with a hydroxyl group on a side chain of the vinyl alcohol polymer include (meth)acrylic acid or derivatives thereof, such as (meth)acrylic acid, (meth)acrylic anhydride, (meth)acrylic acid halide, and (meth)acrylic acid ester. A (meth)acryloyl group can be introduced by subjecting these compounds to an esterification or transesterification reaction in the presence of a base.

Examples of the ethylenically unsaturated group-containing compound to be reacted with a hydroxyl group on a side chain of the vinyl alcohol polymer also include compounds containing an ethylenically unsaturated group and a glycidyl group in the molecule, such as glycidyl (meth)acrylate and allyl glycidyl ether. A (meth)acryloyl group and/or an allyl group can be introduced into the vinyl alcohol polymer by subjecting these compounds to an etherification reaction in the presence of a base.

Further, examples of the ethylenically unsaturated group-containing compound to be reacted with a 1,3-diol group of the vinyl alcohol polymer include compounds containing an ethylenically unsaturated group and an aldehyde group in the molecule, such as acrylaldehyde (acrolein), methacrylaldehyde (methacrolein), 5-norbornene-2-carboxyaldehyde, 7-octenal, 3-vinylbenzaldehyde, and 4-vinylbenzaldehyde. An ethylenically unsaturated group can be introduced into the starting material PVA by subjecting these compounds to an acetalization reaction in the presence of an acid catalyst. More specifically, for example, 5-norbornene-2-carboxyaldehyde, 3-vinylbenzaldehyde, or 4-vinylbenzaldehyde can be subjected to an acetalization reaction to introduce a norbornenyl group or a vinylphenyl group into the starting material PVA. Further, for example, N-(2,2-dimethoxyethyl)(meth)acrylamide can be reacted to introduce a (meth)acryloylamino group into the vinyl alcohol polymer.

In addition to the reactions described above, other methods can also be used to introduce an ethylenically unsaturated group into the vinyl alcohol polymer. Two or more reactions may be used in combination.

Another example of the method for introducing an ethylenically unsaturated group is, for example, a method comprising allowing a compound containing an ethylenically unsaturated group to react with a reactive substituent, such as a carboxy group present in carboxylic acid-modified PVA or an amino group present in amino-modified PVA. To the carboxy group of carboxylic acid-modified PVA, a methacryloyl group can be introduced, for example, by allowing glycidyl methacrylate to react under acidic conditions to form an ester bond. To the amino group of amino-modified PVA, an acryloylamino group can be introduced, for example, by subjecting acrylic anhydride to an amidation reaction in the presence of base, and a vinyloxycarbonyl group can be introduced, for example, by subjecting divinyl adipate to an amidation reaction. In addition to the reactions described above, other methods can also be used to introduce an ethylenically unsaturated group via copolymerized modified PVA. Two or more reactions can be used in combination.

From the viewpoint of ease of production, the polyvinyl alcohol polymer having an ethylenically unsaturated group is preferably a polyvinyl alcohol polymer having an ethylenically unsaturated group introduced via a hydroxyl group on a side chain of the starting material PVA, such as a 1,3-diol group, and is more preferably a vinyl alcohol polymer obtainable by performing an esterification or transesterification reaction of (meth)acrylic acid or a derivative thereof with a hydroxyl group on a side chain of the starting material PVA, or a vinyl alcohol polymer obtainable by performing an acetalization reaction of a compound containing an ethylenically unsaturated group and an aldehyde group in the molecule with a 1,3-diol group of the vinyl alcohol polymer.

### Introduction Rate of Ethylenically Unsaturated Group

From the viewpoint of suppressing embrittlement of the hydrogel, the introduction rate of the ethylenically unsaturated group is preferably 10 mol% or less, more preferably 5 mol% or less, and even more preferably 3 mol% or less, based on all structural units constituting the vinyl alcohol polymer. From the viewpoint of promoting the crosslinking reaction, rapidly forming a hydrogel, and improving the elastic modulus of the resulting hydrogel, the introduction rate of the ethylenically unsaturated group is preferably 0.01 mol% or more, more preferably 0.1 mol% or more, and even more preferably 0.5 mol% or more.

From the viewpoint of improving the mechanical strength of the hydrogel and introducing functional groups different from the hydroxyl groups of PVA, the vinyl alcohol polymer having an ethylenically unsaturated group may further contain a monomer. Examples of the monomer include acrylamides, such as acrylamide, N-isopropylacrylamide, 2-acrylamide-2-methylpropanesulfonic acid, and N,N-dimethylacrylamide; α,β-unsaturated carboxylic acids, such as (meth)acrylic acid, crotonic acid, itaconic acid, maleic acid, and fumaric acid; vinylpyridine; hydroxyethyl (meth)acrylate; styrenesulfonic acid; water-soluble radical-polymerizable monomers, such as polyethylene glycol mono(meth)acrylate; and crosslinking agents having 2 or more ethylenically unsaturated groups in the molecule, such as N,N'-methylenebisacrylamide, ethylene glycol di(meth)acrylate, diethylene glycol di(meth)acrylate, and polyethylene glycol di(meth)acrylate. From the viewpoint of enhancing mechanical strength of the hydrogel, the monomer content is preferably 50 mass% or less, more preferably 30 mass% or less, and even more preferably 10 mass% or less, based on the vinyl alcohol polymer having an ethylenically unsaturated group.

### Crosslinking by Polymerization of Ethylenically Unsaturated Group

The vinyl alcohol polymer having an ethylenically unsaturated group can be gelled by crosslinking the ethylenically unsaturated group introduced into the vinyl alcohol polymer by active energy rays or heat. This provides a crosslinking product of a vinyl alcohol polymer of the present invention. Examples of the active energy rays include gamma rays, UV rays, visible rays, IR rays (heat rays), radio waves, alpha rays, beta rays, electron beams, plasma streams, ionizing rays, and particle beams.

When the vinyl alcohol polymer is crosslinked by heat or ultraviolet rays, visible rays, infrared rays (heat rays), or the like among the active energy rays, the uncrosslinked polymer solution that is described later preferably contains a radical polymerization initiator. Examples of the radical polymerization initiator include a photo-radical polymerization initiator and a thermal radical polymerization initiator.

The photo-radical polymerization initiator is not particularly limited as long as it initiates radical polymerization by irradiation of active energy rays, such as ultraviolet rays and visible rays. From the viewpoint of washing off the photo-radical polymerization initiator after crosslinking, water-soluble polymerization initiators are preferable. Specific examples include 1-[4-(2-hydroxyethoxy)-phenyl]-2-hydroxy-2-methyl-1-propane-1-one (trade name Omnirad 2959, produced by IGM Resins B.V.), α-ketoglutaric acid, phenyl(2,4,6-trimethylbenzoyl) lithium phosphinate (trade name L0290, produced by Tokyo Chemical Industry Co., Ltd.), and Eosin Y.

The thermal radical polymerization initiator is not particularly limited as long as it initiates radical polymerization by heat. Examples include azo initiators, peroxide-based initiators, and the like, which are generally used in radical polymerization. From the viewpoint of improving the transparency and physical properties of the vinyl alcohol polymer, peroxide-based initiators that do not generate a gas are preferable. From the viewpoint of washing off photo-radical polymerization initiators after crosslinking, water-soluble polymerization initiators are preferable. Specific examples include inorganic peroxides, such as ammonium persulfate, potassium persulfate, and sodium persulfate.

Redox polymerization initiators combined with a reducing agent may also be used. When a redox polymerization initiator is used, crosslinking can be carried out by stimulation of mixing of a peroxide initiator with a reducing agent. As the reducing agent to be combined to form a redox polymerization initiator, known reducing agents can be used. Among these, N,N,N',N'-tetramethylethylenediamine, sodium sulfite, sodium bisulfite, sodium hydrosulfite, and the like, which have high water solubility, are preferable.

Azo initiators to be used are also preferably water-soluble azo inhibitors. Specific examples include 2,2'-azobis[2-(2-imidazolin-2-yl)propane]dihydrochloride (trade name VA-044), 2,2'-azobis[2-(2-imidazolin-2-yl)propane]disulfate dihydrate (trade name VA-044B), 2,2'-azobis[2-methylpropionamidine]dihydrochloride (trade name V-50), 2,2'-azobis[N-(2-carboxyethyl)-2-methylpropionamidine]tetrahydrate (trade name VA-057), 2,2'-azobis[2-(2-imidazolin-2-yl)propane] (trade name VA-061), 2,2'-azobis[2-methyl-N-(2-hydroxyethyl)propionamide] (trade name VA-086), and 4,4'-azobis(4-cyanopentanoic acid) (trade name V-501) (all produced by Wako Pure Chemical Corporation).

### Polythiol

When the vinyl alcohol polymer having a vinyl group as the ethylenically unsaturated group is used in the crosslinking step described below, from the viewpoint of promoting crosslinking, for example, a polythiol having two or more thiol groups in the molecule may be added to use a thiol-ene reaction for crosslinking.

The polythiol to be used is preferably a water-soluble polythiol. Examples include polythiols having a hydroxy group, such as dithiothreitol; and polythiols containing an ether bond, such as a terminal thiol product, such as 3,6-dioxa-1,8-octanedithiol, polyethyleneglycoldithiol, and multi-arm polyethyleneglycol.

As described above, the most appropriate method for crosslinking the vinyl alcohol polymer via a covalent bond can be selected as needed. From the viewpoint of the toxicity of the condensing agent and unreacted crosslinking agent contained in the hydrogel, as well as the ease of molding, the method of introducing functional groups into the vinyl alcohol polymer by post-modification and reacting them, especially the method of introducing the ethylenically unsaturated group into the side chain of the vinyl alcohol polymer, is preferred.

### Conjugation of Bioactive Substance

Examples of the bioactive substance include cell-adhesive proteins or peptides, such as gelatin, collagen, laminin, fibronectin, retronectin, vitronectin, elastin, and synthetic RGD peptide; growth factors, such as a fibroblast growth factor (FGF), an epithelial growth factor (EGF), and a vascular endothelial growth factor (VEGF); acidic polysaccharides containing glycosaminoglycan, such as heparin, hyaluronic acid, chondroitin sulfate, dermatan sulfate, or heparan sulfate; antibodies, hormones, serum proteins, antibody-binding proteins, such as albumin, macroglobulin, globulin, and protein A, various pharmaceuticals, and enzymes, such as proteases, lipase, amylase, and cellulase.

The bioactive substance preferably has a functional group capable of binding to the dry hydrogel-forming article. Examples of such functional groups include a primary or secondary amino group, a carboxy group, and a hydroxyl group. The functional group is preferably a primary amino group or a carboxy group, and is more preferably a primary amino group.

### Conjugate

In a first embodiment, the dry hydrogel-forming article of the invention is a conjugate with a bioactive substance. More specifically, in this embodiment, the dry hydrogel-forming article of the present invention may be a dry hydrogel-forming article simply containing a bioactive substance, or may be in the form of a conjugate of a dry hydrogel-forming article and a bioactive substance bound together via a covalent bond. A dry hydrogel-forming article bound via a covalent bond is preferred. When the dry hydrogel-forming article and the bioactive substance are covalently bound, the bioactive substance can be retained in the dry hydrogel-forming article and can stably function.

The method for binding the hydrogel-forming article and the bioactive substance via a covalent bond can be selected from, for example, a method in which a hydroxy group of PVA is activated and is reacted with a functional group of the bioactive substance so as to form a covalent bond, and a method in which carboxylic acid-modified PVA or amino-modified PVA is used to react with a functional group of the bioactive substance so as to form a covalent bond. From the viewpoint of reaction efficiency, the functional group of the bioactive substance is preferably an amino group, a carboxylic acid, or a thiol group, and is more preferably carboxylic acid.

A specific method for activating the hydroxyl group of PVA and introducing a bioactive substance includes, for example, a method using a hydroxyl-activating reagent, such as 1,1'-carbonyldiimidazole, di(N-sucimidyl)carbonate, p-toluenesulphonic acid chloride, 2,2,2-trifluoroethanesulphonic acid chloride, and cyanuric acid chloride. When the hydroxyl group of PVA is reacted with such a hydroxyl-activating agent, a covalent bond can be formed with a primary or secondary amino group of the bioactive substance, a carboxy group, a hydroxyl group, or like functional group. It is also possible to use an acid anhydride reagent, such as succinic anhydride, which can introduce a carboxylic acid by ester bonding to the hydroxyl group of the vinyl alcohol polymer, and an acetal reagent, such as 2,2-dimethoxyethylamine, which can introduce an amino group by acetal bonding to the 1,3-diol group of the vinyl alcohol polymer. Such a carboxylic acid and an amino group can form an amide bond by using, for example, an amino group of the bioactive substance, carboxylic acid, and a carbodiimide condensing agent such as those described above. For example, a reaction of an acid anhydride reagent, such as succinic anhydride, with a crosslinked product of a vinyl alcohol polymer introduces a succinic acid-derived carboxy group (COOH) at the end. This carboxy group and the amino group of the bioactive substance are condensed by using a condensing agent, such as 1,1'-carbonyldiimidazole, dicyclohexylcarbodiimide, or water-soluble carbodiimide, so as to conjugate a bioactive substance to a crosslinked product of a vinyl alcohol polymer via an amide bond.

A specific method for introducing a bioactive substance into carboxylic acid-modified PVA or amino-modified PVA is, for example, using the amino group of a bioactive substance, carboxylic acid, and a carbodiimide condensing agent as mentioned above to form an amide bond. Although the method given above as an example can be used, the most appropriate method can be selected, including other methods, according to the application.

As explained below, the conjugate may be formed after the step of crosslinking the uncrosslinked polymer solution or at the stage of the uncrosslinked polymer solution. In order to avoid reduction or loss of activity of the bioactive substance due to crosslinking, conjugation of the bioactive substance is preferably performed after the crosslinking step.

### Method for Producing Dry Hydrogel-forming Article

The method for producing the hydrogel-forming article of the present invention is not particularly limited. However, it is preferable that first, the hydrogel-forming article of the present invention is produced by preparing an uncrosslinked polymer solution containing the vinyl alcohol polymer (a step of preparing an uncrosslinked polymer solution), then the uncrosslinked polymer solution is molded (a molding step), and subsequently, a vinyl alcohol polymer contained in the uncrosslinked polymer solution is crosslinked to form a gel (a crosslinking step). A specific method is described below.

### Step of Preparing Uncrosslinked Polymer Solution

The step of preparing an uncrosslinked gel solution in the present invention is a step of preparing an uncrosslinked gel solution containing the vinyl alcohol polymer. The uncrosslinked gel solution can be obtained by dissolving the vinyl alcohol polymer in a solvent.

The solvent is preferably water and may further contain a water-soluble organic solvent. Examples of the water-soluble organic solvent include aprotic polar solvents, such as dimethylformamide, dimethylacetamide, dimethylsulfoxide, and N-methylpyrrolidone; monoalcohols, such as methanol, ethanol, propanol, and isopropanol; and polyhydric alcohols, such as ethyleneglycol, diethyleneglycol, triethyleneglycol, and glycerin. The water-soluble organic solvent may be used alone or in a combination of two or more, or as a mixture of the water-soluble organic solvent and water.

When the uncrosslinked polymer solution contains the water-soluble organic solvent, the content is preferably 30 mass% or less, more preferably 20 mass% or less, and even more preferably 10 mass% or less. The content of the solvent in the uncrosslinked polymer solution is preferably 50 mass% or more, more preferably 55 mass% or more, even more preferably 60 mass% or more, and is preferably 99.999 mass% or less, more preferably 99.99 mass% or less, even more preferably 99.9 mass% or less.

The content of the vinyl alcohol polymer in the uncrosslinked polymer solution is preferably 0.001 mass% or more, more preferably 0.01 mass% or more, and even more preferably 0.1 mass% or more. From the viewpoint of suppressing a viscosity increase of the uncrosslinked polymer solution and obtaining good moldability, the content of the vinyl alcohol polymer in the uncrosslinked polymer solution is preferably 50 mass% or less, more preferably 45 mass% or less, and even more preferably 40 mass% or less. When the content of the vinyl alcohol polymer is less than 0.001 mass%, the strength of the obtained gel and the film thickness are too low, whereas when the content of the vinyl alcohol polymer exceeds 50 mass%, the uncrosslinked gel solution has high viscosity, and molding becomes difficult.

### Molding Step

In the production of the hydrogel-forming article of the present invention, the method for molding the uncrosslinked polymer solution is not particularly limited. By using a known method, the uncrosslinked polymer solution can be formed into non-spherical particles, spherical particles, a fine molded article, an article of any shape molded with a 3D printer, a film, thread, hollow fibers, a porous monolith, a coated article, or a like shape.

### Crosslinking Step

The hydrogel-forming article of the present invention is preferably produced by performing a crosslinking step in which a vinyl alcohol polymer is crosslinked after the molding step. The crosslinking in this step can be performed by the method described above. The crosslinking step may be performed by using an uncrosslinked polymer solution that contains a solvent, or may be performed after the solvent in the uncrosslinked polymer solution has been removed. In the crosslinking step, when an uncrosslinked polymer solution that contains a solvent is crosslinked, the resulting gel is highly shrinkable in the drying step described below, which may result in difficulty in forming the gel into a shape such as a film, thread, hollow fibers, coating. In this case, it is preferable that after molding, the uncrosslinked polymer solution is first dried and then crosslinked. The degree of drying of the uncrosslinked polymer solution can be appropriately selected according to the molded product and the crosslinking method. The solvent content is preferably 50% or less, more preferably 25% or less, and even more preferably 10% or less. If the solvent content exceeds 50%, shrinkage tends to increase in the drying step described below.

The uncrosslinked polymer solution may contain components necessary for crosslinking, such as a crosslinking agent and an initiator, as well as the vinyl alcohol polymer before being molded. If the uncrosslinked polymer solution is molded and then dried as described above, the components necessary for crosslinking may be added after molding. The most appropriate crosslinking conditions are selected according to the vinyl alcohol polymer used and the method of crosslinking as described above. Once crosslinking has been completed and a hydrogel is formed, molding becomes difficult. Therefore, molding is preferably performed before a hydrogel is formed. The crosslinking methods described above include methods in which a reaction is started at room temperature or below room temperature only by mixing the uncrosslinked polymer solution and a component necessary for crosslinking. The uncrosslinked polymer solution and the component necessary for crosslinking are preferably mixed just before molding. When crosslinking requires heat, the crosslinking reaction temperature is preferably 100°C or less, more preferably 60°C or less, and even more preferably 37°C or less, from the viewpoint of maintaining the activity of the bioactive substance.

### Conjugation Step

The conjugation step in the present invention is a step of conjugating a bioactive substance to a crosslinked product of a vinyl alcohol polymer to obtain a conjugated hydrogel. Therefore, if a conjugate has been formed at the stage of the uncrosslinked polymer solution as explained above, basically no conjugation step is necessary.

If a carboxylic acid-modified vinyl alcohol copolymer or an amino-modified vinyl alcohol polymer is used as the vinyl alcohol polymer, the bioactive substance can be introduced into the remaining functional group by the conjugation method described above.

It is also possible to first introduce a functional group for conjugation into the hydroxyl group or the like contained in the crosslinked product of the vinyl alcohol polymer after the crosslinking step and then perform a conjugation step. The method for introducing a functional group can be, for example, a method using a hydroxyl-activating agent, an acid anhydride reagent, or an acetal reagent, as described above. This can also introduce a bioactive substance to the functional group by the conjugation method described above.

### Drying Step

The conjugated hydrogel is formed into a dried state by removing water from the target object by a general method, such as hot-air drying, vacuum drying, or freeze drying. The hot-air drying temperature is preferably about 30 to 100°C, and more preferably about 37 to 60°C, in order to avoid reduction or loss of activity of the bioactive substance. The water content of the dry hydrogel-forming article represented by formula (1) below is preferably 75 mass% or less, more preferably 60 mass% or less, even more preferably 50 mass% or less, still even more preferably 40 mass% or less, and particularly preferably 25 mass% or less.

Water content (%) = ( (Weight of water contained in the conjugated hydrogel) / (Dry weight of the conjugated hydrogel + Weight of water contained in the conjugated hydrogel) ) × 100

The appropriate method for measuring the water content of the dry hydrogel-forming article varies depending on the type of solvent contained. When the dry hydrogel-forming article contains only water as a solvent, the water content of the dry hydrogel-forming article can be directly calculated by observing the weight of the dried conjugated hydrogel that remains after evaporating the water contained with a heat-dry moisture meter. When the dry hydrogel-forming article includes a water-soluble organic solvent as described above, the conjugated hydrogel is measured by ¹H-NMR to determine the water content. Specifically, when the conjugated hydrogel is swollen by heavy DMSO, and ¹H-NMR spectrum and integral values are measured, the contents of water and the water-soluble organic solvent can be calculated by comparing the integral values of the peaks derived from the crosslinked product of the vinyl alcohol polymer. This measurement of the water content by ¹H-NMR is useful from the viewpoint that the content of the water-soluble organic solvent and the content of water can be evaluated separately when the hydrogel contains a water-soluble organic solvent. In a preferred embodiment, whether the water-soluble organic solvent is present or not is first confirmed by ¹H-NMR, and when the hydrogel contains only water as a solvent, the water content is measured with a heat-dry moisture meter.

### Sterilization Step

The sterilization in the present invention does not necessarily mean a SAL of 10⁻⁶ or less, as required for pharmaceutical products or medical equipment used for the human body, and may be a SAL of 10⁻³ or less, preferably a SAL of 10⁻⁴ or less, and more preferably a SAL of 10⁻⁵ or less. As long as such a level of sterilization is guaranteed, the sterilized product can be used for testing and research. However, it is preferable to guarantee a SAL of 10⁻⁶ or less when the conjugated hydrogel is incorporated as a part of a pharmaceutical product or medical equipment used for the human body, or the conjugated hydrogel is used in the manufacture of the article.

In a typical embodiment, the conjugated hydrogel is sterilized after the drying step. If the conjugated hydrogel before the drying step, i.e., the conjugated hydrogel containing a large amount of water, is sterilized, the activity of the conjugated bioactive substance is reduced. In contrast, if the sterilization is performed after drying as described above, the activity of the conjugated bioactive substance can be maintained within an acceptable range.

The sterilization method is not particularly limited. Specific examples of sterilization methods usable include the autoclave sterilization method, the ethylene oxide gas sterilization method, the hydrogen peroxide low-temperature plasma sterilization method, the dry heat sterilization method, the chemical sterilization method with glutaraldehyde or the like, and the radiation sterilization method with gamma rays or electron beams. Among these, from the viewpoint of ease of maintaining the activity of the conjugated bioactive substance, the ethylene oxide gas sterilization method, the hydrogen peroxide low-temperature plasma sterilization method, and the radiation sterilization method are preferable. From the viewpoint of absence of residues, the radiation sterilization method is more preferred. The dry hydrogel-forming article of the present invention is preferably free of ethylene oxide gas, free of glutaraldehyde, and free of hydrogen peroxide.

The sterilization is preferably performed by placing a dry hydrogel-forming article in a container, sealing the container, and then performing the sterilization step. The container to be used can be a flexible resin container, such as a pouch made of resin. Sealing can be done by heat sealing. In addition to this, a bottle container with a lid that can be opened after sterilization can be used as the container, and sealing can be done by screwing down the lid or heat sealing like an ampoule. For example, when radiation sterilization is to be performed, a container made of a material that is radiotransparent and that maintains sufficient strength after radiation exposure is preferably used. Examples of the material of the container include polyolefin resins, such as polyethylene, polypropylene, and polybutylene; copolymers of two or more olefins; polyester resins, such as polyethylene terephthalate and polybutylene terephthalate; vinyl resins, such as polyvinyl alcohol, a saponified product of an ethylene-vinyl acetate copolymer, polyvinyl chloride, and polyvinylidene chloride; and polycarbonate resins, polystyrene resins, silicone resins, polyamide resins, and glass. The container may be composed of a single layer or a laminate of such materials. Examples of usable materials include such materials on which aluminum, silica, or the like are vapor-deposited, metal foils, such as aluminum films and aluminum-laminated films, and multilayer films containing metal foil. It is also preferable to sterilize the hydrogel-forming article in a dry state.

Examples of the method for performing sterilization validation include the half-cycle method, the overkill method, the combined bioburden/BI method, and the absolute bioburden method, any of which can be used. In the case of radiation sterilization, sterilization validation according to the absolute bioburden method described in ISO (ISO 11137-2:2013) or JIS (JIS T 0806-2:2014) is performed. The methods for setting the sterilization dose in the sterilization validation include Method 1, the VDₘₐₓ method, and Method 2, all of which can be used to sterilize the dry hydrogel-forming article of the present invention. Method 1 and the VDₘₐₓ method are methods of setting the sterilization dose based on the number of bacteria adhering to the dry hydrogel-forming article. Method 2 is a method of setting the sterilization dose based on the radioresistance of the bacteria adhering to the dry hydrogel-forming article. To determine the sterilization dose using Method 1 and the VDₘₐₓ method, the bioburden test as described in, for example, ISO 11737-1:2006 or JIS T11737-1:2013 can be performed to determine the number of bacteria adhering to the dry hydrogel-forming article before sterilization can be measured. In view of the amount of data and costs required, Method 1 and the VDₘₐₓ method are preferred. In view of cost reduction, the VDₘₐₓ method is more preferable.

To achieve a SAL of 10⁻⁶ or less, an irradiation dose of at least 11 kilo Gray (kGy) is required in Method 1, an irradiation dose of at least 15 kGy is required in the VDₘₐₓ method, and an irradiation dose of at least 8.2 kGy is required in Method 2. It was common knowledge that the conjugated hydrogel cannot withstand this very high energy gamma ray irradiation dose. The present invention according to a preferred embodiment has been achieved based on the finding that when a dried conjugated hydrogel-forming article is used, damage to the product by gamma ray irradiation with an energy as high as 8.2 kGy or more can be prevented, and a sterilized conjugated hydrogel-forming article that maintains the bioactive substance activity within the desired range can be produced.

The dry hydrogel-forming article of the present invention may be used in combination with cells or may contain cells. When the dry hydrogel-forming article of the present invention is used in combination with cells or contains cells, water is preferably supplied to the article to form a hydrogel. The term "cell" as referred to in the present specification preferably includes, but is not limited to, multipotent stem cells, tissue stem cells, somatic cells, and mammal-derived established cell lines for use in producing a useful substance for a pharmaceutical or the like or in therapy etc., and insect cells.

Cells include adherent cells and floating cells. Adherent cells are cells that grow by adhering to a carrier, such as the hydrogel of the present invention, in cell culture. Floating cells are cells that basically do not require adhesion to a carrier in cell proliferation. Floating cells include cells that can weakly adhere to a carrier.

The multipotent stem cells are stem cells having an ability to differentiate into cells of any tissue (pluripotent differentiation). Examples include embryo stem cells (ES cells), induced pluripotent stem cells (iPS cells), embryonic germ stem cells (EG cells), and germ stem cells (GS cells).

The tissue stem cells mean stem cells having an ability to differentiate into various cell species although the differentiable tissue is limited (pluripotent differentiation). Examples of tissue stem cells include undifferentiated bone marrow mesenchymal stem cells, skeletal muscle stem cells, hematopoietic stem cells, neural stem cells, hepatic stem cells, adipose tissue stem cells, epidermal stem cells, intestinal stem cells, sperm stem cells, pancreatic stem cells (pancreatic duct epithelial stem cells), leukocytic stem cells, lymphocytic stem cells, and corneal stem cells.

The somatic cells indicate cells to constitute multicellular organisms. Examples of somatic cells include, but are not limited to, osteoblast cells, chondrocytes, hematopoietic cells, epithelial cells (e.g., mammary gland epithelial cells), endothelial cells (e.g., vascular endothelial cells), epidermal cells, fibroblasts, mesenchyme-derived cells, cardiac muscle cells, myoblastic cells, smooth myocytes, skeletal myocytes derived from living organisms, human tumor cells, fiber cells, EB virus-modified cells, hepatocytes, renal cells, bone marrow cells, macrophages, hepatic parenchymal cells, small intestinal cells, mammary cells, salivary gland cells, thyroid gland cells, skin cells, plasma cells, T cells, B cells, killer cells, lymphoblasts, and pancreatic beta cells.

The mammal-derived established cell lines include CRFK cells, 3T3 cells, A549 cells, AH130 cells, B95-8 cells, BHK cells, BOSC23 cells, BS-C-1 cells, C3H10T1/2 cells, C-6 cells, CHO cells, COS cells, CV-1 cells, F9 cells, FL cells, FL5-1 cells, FM3A cells, G-361 cells, GP+E-86 cells, GP+envAm12 cells, H4-II-E cells, HEK293 cells, HeLa cells, HEp-2 cells, HL-60 cells, HTC cells, HUVEC cells, IMR-32 cells, IMR-90 cells, K562 cells, KB cells, L cells, L5178Y cells, L-929 cells, MA104 cells, MDBK cells, MDCK cells, MIA PaCG-2 cells, N18 cells, Namalwa cells, NG108-15 cells, NRK cells, OC10 cells, OTT6050 cells, P388 cells, PA12 cells, PA317 cells, PC-12 cells, PER.C6 cells, PG13 cells, QGH cells, Raji cells, RPMI-1788 cells, SGE1 cells, Sp2/O-Ag14 cells, ST2 cells, THP-1 cells, U-937 cells, V79 cells, VERO cells, WI-38 cells, ϕ2 cells, and ϕCRE cells.

The insect cells include silkworm cells (e.g., BmN cells and BoMo cells), *Bombyx mandarina* cells, tussah cells, ailanthus moth cells, cabbage armyworm cells (e.g., Sf9 cells and Sf21 cells), *Lemyra imparilis* cells, leaf roller cells, vinegar fly cells, *Boettcherisca peregrine* cells, *Aedes albopictus* cells, *Papilio xuthus* cells, American cockroach cells, and *Trichoplusia ni* cells (e.g., Tn-5 cells, High Five cells and MG1 cells).

The above cells may aggregate together or may be differentiated. The aggregated cells may have a function as an organ. The cells may be those immediately after being collected from living bodies, or may be cultivated. The cells collected from living bodies may form an organ.

When the dry hydrogel-forming article is formed into a hydrogel and, for example, brought into contact with cells to culture the cells, it is preferable to add a medium component. The medium used is not particularly limited and can be freely selected to suit the cells used. Basic media, such as DMEM, MEM and F12, containing fetal bovine serum, serum-reduced media, and serum-free media can be used. Examples of serum-free media usable include xeno-free media and complete synthetic media that are free of serum and that contain neither protein components nor animal components.

The dry hydrogel-forming article according to the present invention can provide a sterilized conjugated hydrogel-forming article that has excellent hydrophilicity, reactivity, biodegradability, and biocompatibility, as well as low toxicity and high flexibility and strength without using an expensive process, such as a sterile environment. Therefore, the dry hydrogel-forming article of the present invention is useful as a carrier or a support. The carriers comprising the dry hydrogel-forming article of the invention can be suitably used for applications such as enzyme immobilization carriers, affinity carriers, cell culture carriers, and drug delivery carriers.

### 2. Hydrogel

In a second embodiment, the present invention provides a hydrogel comprising a crosslinked product of a vinyl alcohol polymer having an ethylenically unsaturated group and a carboxy group and a bioactive substance having an amino group, wherein the carboxy group of the crosslinked product and the amino group of the bioactive substance are covalently bonded via an amide bond. The details, production method, use method, etc. in the second embodiment can be the same conditions as those in the first embodiment of the present invention described in "1. Dry Hydrogel-forming Article," unless specifically stated otherwise herein.

### Hydrogel

In this embodiment, the hydrogel of the present invention is a conjugated hydrogel or a dried product thereof, or a molded article thereof, the conjugated hydrogel being obtained by conjugating a bioactive substance having an amino group to a crosslinked product of a vinyl alcohol polymer having an ethylenically unsaturated group and a carboxy group. The hydrogel of the present invention may contain water or may be in a dried state. The dried hydrogel swells upon contact with an aqueous solution, such as water, a buffer, body fluid, or a culture medium to form a hydrogel. The form of the molded article includes, for example, general non-spherical particles, spherical particles, a film, thread, hollow fibers, and a porous monolith.

In the present specification, the "amide bond-conjugated hydrogel" means a hydrogel in which a bioactive substance having an amino group is covalently bound to a crosslinked product of the vinyl alcohol polymer having an ethylenically unsaturated group and a carboxy group via an amide bond to the carboxy group of the vinyl alcohol polymer.

Further, the form of the molded particle, as well as its dimensions, production method, etc., can be, for example, the same as those described above in "1. Dry Hydrogel-forming Article."

In the second embodiment, the hydrogel of the present invention may be in a dry state from which a solvent has been removed, or may contain a solvent. The solvent is preferably water and may further contain a water-soluble organic solvent. Examples of the water-soluble organic solvent include aprotic polar solvents, such as dimethylformamide, dimethylacetamide, dimethyl sulfoxide, and N-methylpyrrolidone; monoalcohols, such as methanol, ethanol, propanol, and isopropanol; and polyalcohols, such as ethylene glycol, diethylene glycol, triethylene glycol, and glycerin. Such water-soluble organic solvents can be mixed and used. The content of the solvent in the hydrogel is preferably 0.1 to 99 mass%, preferably 0.5 to 98 mass%, and preferably 1 to 97 mass%.

### Method for Producing Vinyl Alcohol Polymer

The method for producing the vinyl alcohol polymer, raw materials, viscosity measurement method, etc. in the second embodiment of the present invention can be the same as those in the first embodiment of the present invention described in "1. Dry Hydrogel-forming Article," unless specifically stated otherwise herein.

### Crosslinking Product of Vinyl Alcohol Polymer

In the second embodiment, the hydrogel of the present invention includes a crosslinked product of a vinyl alcohol polymer having an ethylenically unsaturated group and a carboxy group. The total amount of the vinyl alcohol-derived structural unit and the vinyl ester-derived structural unit relative to all structural units constituting the vinyl alcohol polymer used in the present invention is preferably 80 mol% or more, more preferably 90 mol% or more, and even more preferably 95 mol% or more.

### Vinyl Alcohol Polymer with Ethylenically Unsaturated Group

In the second embodiment, the vinyl alcohol polymer of the present invention has an ethylenically unsaturated group covalently bound thereto as a crosslinkable moiety. The ethylenically unsaturated group allows a polymerization reaction to proceed under mild conditions, even in water, as described below, and enables the vinyl alcohol polymer to be crosslinked and gelled. The ethylenically unsaturated group is extremely useful because it can be used in various molding processs, including molding with 3D printers.

The ethylenically unsaturated group is not particularly limited and can be freely selected. The ethylenically unsaturated group is preferably a group capable of forming a crosslink between vinyl alcohol polymer chains by active energy rays, heat, a redox polymerization initiator, or the like as described below. The ethylenically unsaturated group is preferably a radically polymerizable group. Examples of radically polymerizable groups include cyclic unsaturated hydrocarbon groups, such as a vinyl group, a (meth)acryloyl group, a (meth)acryloylamino group, a vinylphenyl group, a cyclohexenyl group, a cyclopentenyl group, a norbornenyl group, and a dicyclopentenyl group, and derivatives thereof. These ethylenically unsaturated groups may be present on either a side chain or a terminal of a vinyl alcohol polymer chain.

The vinyl group in the present invention includes not only an ethenyl group, but also a chain unsaturated hydrocarbon group, such as an allyl group or an alkenyl group, a vinyloxycarbonyl group, and the like.

Among the radically polymerizable groups, from the viewpoint of enhancing the mechanical strength of the hydrogel particles, at least one member selected from the group consisting of a vinyl group, a (meth)acryloyl group, a (meth)acryloylamino group, a vinylphenyl group, a norbornenyl group, and derivatives thereof is preferable. Further, from the viewpoint of reactivity, a functional group having a terminal unsaturated carbon bond is preferable, and a (meth)acryloyl group is more preferable.

The introduction of the ethylenically unsaturated group into the vinyl alcohol polymer is preferably performed, for example, via a side chain or a terminal functional group of the vinyl alcohol polymer. It is more preferable that a compound containing an ethylenically unsaturated group (also simply referred to below as an "ethylenically unsaturated group-containing compound") is allowed to react with a hydroxy group in the side chain of the vinyl alcohol polymer.

Examples of compounds containing an ethylenically unsaturated group that is allowed to react with a hydroxy group in the side chain of the vinyl alcohol polymer include (meth)acrylic acids, such as (meth)acrylic acid, (meth)acrylic acid anhydride, (meth)acrylic acid halide, and (meth)acrylic acid ester or derivatives thereof; and norbornene derivatives, such as 5-norbornene-2,3-dicarboxylic anhydride and 5-norbornene-2-carboxylic acid. These compounds may be subjected to an esterification reaction or a transesterification reaction in the presence of a base so as to introduce a (meth)acryloyl group and a norbornene group.

In addition to these, examples of the ethylenically unsaturated group-containing compound that is allowed to react with a hydroxy group in the side chain of the vinyl alcohol polymer include compounds containing an ethylenically unsaturated group and a glycidyl group in a molecule. Specific examples include glycidyl (meth)acrylate and allyl glycidyl ether. These compounds may be subjected to an etherification reaction in the presence of a base so as to introduce a (meth)acryloyl group or an allyl group into the vinyl alcohol polymer.

Further, the compound containing an ethylenically unsaturated group that is allowed to react with the 1,3-diol group of the vinyl alcohol polymer, the method for introducing an ethylenically unsaturated group into the vinyl alcohol polymer, and the vinyl alcohol polymer having an ethylenically unsaturated group can be, for example, the same as those described in "1. Dry Hydrogel-forming Article."

### Introduction Rate of Ethylenically Unsaturated Group

The introduction rate of the ethylenically unsaturated group and the monomer that the vinyl alcohol polymer having an ethylenically unsaturated group may further contain are, for example, the same as those described in "1. Dry Hydrogel-forming Article."

### Crosslinking by Polymerization of Ethylenically Unsaturated Group

Being able to obtain a crosslinked product of a vinyl alcohol polymer having an ethylenically unsaturated group by crosslinking the ethylenically unsaturated group introduced into the vinyl alcohol polymer by active energy rays or heat to form a gel; examples of active energy rays usable in the crosslinking, and examples of radical polymerization initiators (e.g., radical photopolymerization initiators, thermal radical polymerization initiators, redox polymerization initiators, azo initiators, and the like in combination with a reducing agent) can be the same as those described in "1. Dry Hydrogel-forming Article."

### Polythiol

In the crosslinking step, when the vinyl alcohol polymer having a vinyl group as the ethylenically unsaturated group is used, from the viewpoint of promoting curing, for example, a polythiol having two or more thiol groups in the molecule may be added to allow crosslinking to proceed by using a thiol-ene reaction, and examples of the polythiol usable in the crosslinking can be, for example, the same as those mentioned in "1. Dry Hydrogel-forming Article."

### Vinyl Alcohol Polymer Having Carboxy Group

In the second embodiment, the vinyl alcohol polymer of the present invention has a carboxy group. The method for introducing a carboxy group into the vinyl alcohol polymer can be selected, for example, from a method comprising activating the hydroxyl group of PVA to introduce a carboxy group, and a method directly using the carboxy group contained in carboxylic acid-modified PVA into which a monomer having a carboxy group other than the vinyl ester monomer has been introduced in advance by copolymerization in production of the vinyl alcohol polymer.

The method for introducing the carboxy group by activating the hydroxyl group of PVA includes, for example, using an acid anhydride reagent, such as succinic anhydride, which is capable of introducing a carboxylic acid into the hydroxyl group by ester bonding, and using an acetal reagent, such as glyoxylic acid, which is capable of introducing a carboxy group into the 1,3-diol group of the vinyl alcohol polymer by acetal bonding. An amide bond can be formed between such a carboxylic acid (COOH) and the amino group of a bioactive substance by using a condensing agent used in peptide synthesis, such as carbonyldiimidazole, dicyclohexylcarbodiimide, and water-soluble carbodiimide.

Examples of the carboxy-containing monomer that is other than the vinyl ester monomer constituting the carboxylic acid-modified PVA include α,β-unsaturated carboxylic acids, such as (meth)acrylic acid, maleic acid, fumaric acid, and itaconic acid; (meth)acrylic acid alkyl esters, such as methyl (meth)acrylate and ethyl (meth)acrylate; α,β-unsaturated carboxylic acid anhydrides, such as maleic anhydride, and itaconic anhydride, and derivatives thereof. The vinyl ester monomer is copolymerized with a carboxy-containing monomer that is other than the vinyl ester monomer constituting the carboxylic acid-modified PVA, and is then saponified to produce a carboxylic acid-modified vinyl alcohol polymer (a carboxylic acid-modified

### PVA) .

From the viewpoint of keeping swelling of the hydrogel in a solvent (in particular, water) moderate and maintaining physical properties, such as gel strength, the introduction rate of the carboxy group is preferably 50 mol% or less, more preferably 30 mol% or less, and even more preferably 15 mol% or less, based on all structural units comprising the vinyl alcohol polymer. From the viewpoint of keeping the conjugation density of the bioactive substance high and expressing high functionality, the introduction rate of the carboxy group is preferably 0.1 mol% or more, more preferably 0.5 mol% or more, and even more preferably 1.0 mol% or more.

### Bioactive Substance

Examples of the bioactive substance having a (primary or secondary) amino acid include cell adhesion proteins or peptides, such as gelatin, collagen, laminin, fibronectin, and synthetic RGD peptide; growth factors, such as a fibroblast growth factor (FGF), an epithelial growth factor (EGF), and a vascular endothelial growth factor (VEGF); antibody-binding proteins, such as antibodies, serum proteins, albumin, macroglobulin, globulin, and protein A; glycosaminoglycans, such as heparin, hyaluronic acid, chondroitin sulfate, dermatan sulfate, and heparan sulfate; various medical products; and enzymes, such as protease, lipase, amylase, and cellulase. These bioactive substances contain an amino group, which can be used to conjugate a bioactive substance via an amide bond. The bioactive substance is preferably a bioactive polypeptide containing two or more amino acids. The amino group can be either an aromatic or aliphatic amino group, and is preferably an aliphatic amino group. The side-chain amino group or the N-terminal amino group of lysine (Lys) in the bioactive polypeptide is preferred. The amino group contained in the bioactive substance can be either a primary or secondary amino group. The bioactive substance contains either one or both of these types of amino groups. The primary or secondary amino groups contained in the bioactive substance can both form an amide bond with the carboxy group of the crosslinking product of the vinyl alcohol polymer.

### Conjugate

In the second embodiment, the hydrogel of the present invention is a conjugate of a crosslinked product of a vinyl alcohol polymer having an ethylenically unsaturated group and a carboxy group, and a bioactive substance having an amino group. More specifically, the hydrogel of the present invention is a hydrogel in which a bioactive substance having an amino group is bound to the carboxy group of a vinyl alcohol polymer via a covalent bond (-CO-NH-). The formation of a covalent bond (amide bond) between the hydrogel and the bioactive substance enables the bioactive substance to be retained in the hydrogel and function stably.

A specific method for conjugating a bioactive substance to a crosslinked product of a vinyl alcohol polymer can be, for example, a method of covalently bonding the amino group of a bioactive substance and the carboxy group of the vinyl alcohol polymer via an amide bond by using a carbodiimide condensing agent, as described above. The optimum method can be used, including other methods, depending on the application.

The conjugate with a bioactive substance can be formed after the step of crosslinking the uncrosslinked gel solution as explained below, or a bioactive substance can be conjugated at the stage of the uncrosslinked gel solution.

### Method for Producing Hydrogel

The method for producing the hydrogel of the present invention is not particularly limited. However, the hydrogel of the present invention is preferably produced by first performing the step of preparing an uncrosslinked gel solution containing the vinyl alcohol polymer (step of preparing an uncrosslinked gel solution), then performing the step of molding the uncrosslinked polymer solution (a molding step), and subsequently performing the step of crosslinking the vinyl alcohol polymer contained in the uncrosslinked gel solution to form a gel (a crosslinking step). The step of conjugating a bioactive substance can be performed before or after the crosslinking step.

A specific method is described below.

### Step of Preparing Uncrosslinked Gel Solution

The step of preparing an uncrosslinked gel solution in the present invention is a step of preparing an uncrosslinked gel solution containing the vinyl alcohol polymer. The uncrosslinked gel solution can be obtained by dissolving the vinyl alcohol polymer in a solvent.

The type and amount of solvent used in the method, the content of the vinyl alcohol polymer in the uncrosslinked gel solution, etc. can be the same conditions as those in the first embodiment of the invention described in "1. Dry Hydrogel-forming Article."

### Molding Step

In the second embodiment, in the production of the hydrogel, the method for molding the uncrosslinked gel solution is not particularly limited, and the method described in "1. Dry Hydrogel-forming Article" can be used as appropriate.

### Crosslinking Step

In the second embodiment, the hydrogel of the present invention is preferably produced by performing the crosslinking step of crosslinking the vinyl alcohol polymer after the molding step. The details of the crosslinking step can be the same conditions as those in the first embodiment of the present invention described in "1. Dry Hydrogel-forming Article."

### Conjugation Step

When a conjugate has been formed at the stage of the uncrosslinked gel solution as explained above, the conjugation step after the crosslinking step is basically unnecessary. When a carboxylic acid-modified PVA is used as the vinyl alcohol polymer, a bioactive substance can be introduced into the remaining functional group by the conjugation method described above.

It is also possible to first introduce a functional group for conjugation (a carboxy group) into the hydroxyl group or the like contained in the hydrogel after the crosslinking step and then perform a conjugation step. The method for introducing a functional group can be, for example, a method using a hydroxyl-activating reagent, an acid anhydride reagent, or an acetal reagent, as described above. This can also introduce a bioactive substance having an amino group into the carboxy group by the conjugation method described above.

In the second embodiment, the hydrogel of the present invention may be used in combination with cells or may contain cells. The details of the cells, culture method, etc. in the second embodiment are the same as those described in "1. Dry Hydrogel-forming Article."

In the second embodiment, the hydrogel of the present invention has excellent hydrophilicity, reactivity, biodegradability, and biocompatibility, as well as high flexibility and strength, and has a bioactive substance or an enzyme efficiently introduced thereinto via a covalent bond by using a crosslinking method with low toxicity. Therefore, the hydrogel of the present invention is useful as a carrier or a support. The carriers comprising the hydrogel of the invention can be suitably used for applications such as enzyme immobilization carriers, affinity carriers, cell culture carriers, and drug delivery carriers.

### Examples

The present invention is described in more detail below with reference to Examples. However, the invention is not limited to these Examples.

### Starting Materials for Use

The main components used in Synthesis Examples, Examples, and Comparative Examples are listed below.

### Starting Material PVA

- PVA117: Polyvinyl alcohol (trade name: PVA117, degree of polymerization: 1700, degree of saponification: about 98.0 to 99.0 mol%, viscosity (4%, 20°C): 25.0 to 31.0 mPa·s, produced by Kuraray Co., Ltd.

The degree of polymerization of starting material PVA was measured according to JIS K 6726:1994.
- AF-17: Polyvinyl alcohol (trade name: AF-17, 1.3 mol% itaconic acid copolymerization (Scientific Reports, 2017, Vol. 7, p. 45146), degree of saponification: 96.5 mol% or more, viscosity (4%, 20°C): 30 ± 3 mPa·s, produced by Japan Vam & Poval Co., Ltd.)

### Compound Having Ethylenically Unsaturated Group

- Vinyl methacrylate: Tokyo Chemical Industry Co., Ltd.
- 5-Norbornene-2-carboxyaldehyde: Tokyo Chemical Industry Co., Ltd.

### Synthetic Reagent

- 50% glutaraldehyde solution: Fujifilm Wako Pure Chemical Corporation
- Triethylamine: produced by Fujifilm Wako Pure Chemical Corporation
- Succinic anhydride: F produced by Fujifilm Wako Pure Chemical Corporation
- Liquid paraffin: produced by Fujifilm Wako Pure Chemical Corporation
- Span 80: produced by Fujifilm Wako Pure Chemical Corporation

### Radical Polymerization Initiator

- Potassium persulfate: produced by Fujifilm Wako Pure Chemical Corporation
- L0290: Lithium phenyl(2,4,6-trimethylbenzoyl)phosphinate (photo-radical polymerization initiator, trade name: L0290, Tokyo Chemical Industry Co., Ltd.)

### Polythiol

- 3,6-Dioxa-1,8-octanedithiol: produced by Tokyo Chemical Industry Co., Ltd.

### Activation Reagent for Hydroxy Groups

- 1,1'-Carbonyldiimidazole: produced by Tokyo Chemical Industry Co., Ltd.
- Succinic anhydride: Produced by Tokyo Chemical Industry Co., Ltd., or by Fujifilm Wako Pure Chemical Corporation Conjugate-forming Reagent
- 1-(3-dimethylaminopropyl)-3-ethylcarbodiimide hydrochloride: produced by Fujifilm Wako Pure Chemical Corporation
- N-hydroxysuccinimide: produced by Tokyo Chemical Industry Co., Ltd.

### Bioactive Substance

- Gelatin (pig origin, Type A): Produced by Sigma-Aldrich Japan
- Collagen (trade name: Cellmatrix, Type I-C): produced by Nitta Gelatin Inc.

### Solvent

- Ion-exchanged water: Ion-exchanged water with an electrical conductivity of 0.08 × 10⁻⁴ S/m or less
- PBS: Prepared by dissolving PBS tablets (produced by Takara Bio Inc.) in a predetermined amount of ion-exchanged water
- MES buffer: A 0.1 mol/L aqueous solution of 2-morpholinoethanesulfonic acid monohydrate (produced by Dojindo Laboratories) was prepared and neutralized with a 0.1 mol/L aqueous solution of NaOH to a pH of 5.6.

### Method for Measuring Compounds Synthesized in Synthesis Examples Introduction Rate of Ethylenically Unsaturated Groups and Carboxy Groups (Succinic Acid)

The introduction rate of the ethylenically unsaturated group and carboxy group (succinic acid) into the vinyl alcohol polymers having an ethylenically unsaturated group obtained in the following Synthesis Examples was measured by ¹H-NMR. The introduction rate is determined from the ratio of the integral of the signal of the ethylenically unsaturated group or the carboxy group (methylene succinate group) to the signal of a vinyl alcohol polymer.

Similarly, ¹H-NMR measurement can be performed for hydrogel particles and sheets swollen in a deuterated solvent, and the introduction rate in the hydrogel particles and sheets is determined from the ratio of the integral of the signal of the carboxy group (methylene succinate group) to the signal of a vinyl alcohol polymer.

### ¹H-NMR Measurement Conditions

Equipment: JNM-ECX400 nuclear magnetic resonator, produced by JEOL Ltd.
Temperature: 25°C

### Water Content

The water content in the conjugated hydrogels obtained in the Examples below was measured with a heat-dry moisture meter when only water was present as a solvent, and measured by ¹H-NMR when a water-soluble organic solvent was present.

### Conditions for Measurement with Heat-dry Moisture Meter

Equipment: Heat-dry moisture meter, produced by A&D Company, Limited

### ¹H-NMR Measurement Conditions

The water content can be calculated by ¹H-NMR (deuterated DMSO solvent). The water content is determined from the ratio of the integral of the signal of water (3.3 ppm) to the signal of the vinyl alcohol polymer. Water originally contained in the deuterated DMSO solvent is subtracted.
Equipment: JNM-ECX400 nuclear magnetic resonator, produced by JEOL Ltd.
Temperature: 25°C

### Synthesis Examples

### Synthesis Example 1-1: Synthesis of Vinyl Alcohol Polymer Having Ethylenically Unsaturated Group

40 g (monomer repeating unit: 908 mmol) of PVA117 (starting material PVA) was placed in a separable flask equipped with a 1-L Dimroth condenser. 350 mL of dimethylsulfoxide (DMSO) was added, and stirring was started with a mechanical stirrer. After the temperature was raised to 80°C by a water bath, stirring was continued at 80°C for four hours. After the starting material PVA was visually confirmed to have dissolved, 2.1 g (18.7 mmol) of vinyl methacrylate was added and stirred with heating at 80°C and stirred for another 3 hours at 80°C. After cooling, the reaction solution was poured into 2 L of methanol with stirring. Stirring was ended, and the mixture was allowed to stand for one hour. The obtained solid was collected and further washed by immersion in 1 L of methanol for one hour. This washing operation was performed a total of three times. The collected solid was vacuum-dried overnight at room temperature to obtain methacryloylated PVA117. The introduction rate of an ethylenically unsaturated group (methacryloyl group) into the methacryloylated PVA117 was 2.0 mol% relative to the repeating unit in the starting material PVA (simply referred to as "MA-PVA117 (2.0)" below).

### Synthesis Example 1-2

60 g (monomer repeating unit: 1.36 mol) of PVA117 (starting material PVA) was placed in a separable flask equipped with a 1-L Dimroth condenser. 540 mL of ion-exchanged water was added, and stirring was started with a mechanical stirrer. After the temperature was raised to 80°C by a water bath, stirring was continued at 80°C for four hours. After the starting material PVA was visually confirmed to have dissolved, the temperature was lowered to 40°C. While the solution was stirred at 40°C, 2.5 g (20.5 mmol) of 5-norbornene-2-carboxyaldehyde and 22 mL of a 10 vol% aqueous solution of sulfuric acid were added, and the mixture was further stirred at 40°C for four hours. After cooling, the solution was neutralized by adding 80 mL of a 1N aqueous solution of NaOH and desalted in a dialysis membrane with a fractional molecular weight of 3,500 (performed four times for 5 L of ion-exchanged water). The desalted aqueous solution was poured into 2 L of methanol with stirring and allowed to stand for one hour. The obtained solid was collected and further washed by immersion in 1 L of methanol for one hour. The collected solid was vacuum-dried overnight at room temperature, thereby obtaining norbornene-introduced (Nor) PVA117. The introduction rate of an ethylenically unsaturated group (norbornenyl group) into the norbornene-introduced PVA117 was 1.3 mol% relative to the repeating unit in the starting material PVA ("Nor-PVA117 (1.3)" below).

### Synthesis Example 1-3

10 g (monomer repeating unit: 220 mmol) of MA-PVA117 (2.0) prepared in Synthesis Example 1-1 was placed in a separable flask equipped with a 0.5-L Dimroth condenser. 90 mL of dimethylsulfoxide (DMSO) was added, and stirring was started with a mechanical stirrer. After the temperature was raised to 80°C by a water bath, stirring was continued at 80°C for four hours. After the starting material PVA was visually confirmed to have dissolved, the water bath was set to 60°C. After the internal temperature was confirmed to have reached 60°C, 1.2 g (12.1 mmol) of triethylamine and 1.1 g (11 mmol) of succinic anhydride were added, and the mixture was further stirred at 60°C for five hours. After cooling, the reaction solution was poured into 0.5 L of methanol with stirring. Stirring was ended, and the mixture was allowed to stand for one hour. The obtained solid was collected and further washed by immersion in 0.5 L of methanol for one hour. This washing operation was performed a total of three times. The collected solid was vacuum-dried overnight at room temperature, thereby obtaining succinic acid-introduced methacryloylated PVA117. The introduction rate of succinic acid (SA) was 3.4 mol% relative to the repeating unit of MA-PVA117 (2.0) ("MA-PVA117 (2.0)-SA (3.4)" below).

### Production of Molded Article: Particles and Sheets

### Synthesis Example 1-A: Particles

440 mL of ion-exchanged water was added to 60 g of MA-PVA117 (2.0), and the mixture was stirred at 80°C for four hours to dissolve MA-PVA117 (2.0). After cooling to room temperature, potassium persulfate, which is a water-soluble thermal radical polymerization initiator, was added to give 0.1 mass% to the aqueous solution of MA-PVA117 (2.0), thereby preparing an uncrosslinked polymer solution.

3300 mL of liquid paraffin and 8 g of Span 80 were placed in a separable flask equipped with a 5-L Dimroth condenser, and then the uncrosslinked polymer solution was slowly added thereto. The mixture was stirred at 350 rpm with a mechanical stirrer to form a W/O dispersion. After the temperature was raised to 40°C by a water bath, purging with nitrogen was performed for 30 minutes. Thereafter, stirring was continued at 70°C for three hours. After cooling to room temperature, the liquid paraffin in which hydrogel particles were dispersed was filtered through a mesh with an opening of 100 µm. The obtained hydrogel particles were washed with 3 L of hexane in total to remove the liquid paraffin. The obtained hydrogel particles were then classified into particle sizes of 180 to 300 µm by using JIS standard sieves. The hydrogel particles were further added to 1 L of acetone, dehydrated, and then dried under reduced pressure, thereby obtaining dry hydrogel particles ("MA-PVA117 (2.0) gel particles" below).

### Synthesis Example 1-B: Particles

440 mL of ion-exchanged water was added to 60 g of Nor-PVA117 (1.3), and the mixture was stirred at 80°C for four hours to dissolve Nor-PVA117 (1.3). After cooling to room temperature, 3.4 g of 3,6-dioxa-1,8-octanedithiol (polythiol) was added to the aqueous solution of Nor-PVA117 (1.3) and stirred. Potassium persulfate, which is a water-soluble thermal radical polymerization initiator, was added to this solution to give 0.1 mass% and dissolved to prepare an uncrosslinked polymer solution. Dry hydrogel particles were obtained by using the uncrosslinked polymer solution according to the same method as in Synthesis Example 1-A ("Nor-PVA117 (1.3) gel particles" below).

### Synthesis Example 1-C: Sheet

44 mL of ion-exchanged water was added to 6 g of MA-PVA117 (2.0)-SA (3.4), and the mixture was stirred at 80°C for four hours to dissolve MA-PVA117 (2.0)-SA (3.4). After cooling to room temperature, potassium persulfate, which is a water-soluble thermal radical polymerization initiator, was added to the aqueous solution of MA-PVA117 (2.0)-SA (3.4) to give 0.1 mass% and dissolved to prepare an uncrosslinked polymer solution. The uncrosslinked polymer solution was poured into the space between two glass plates with a 0.5-mm spacer between them in a nitrogen atmosphere and cured at 40°C for 3 hours. The cured 0.5-mm-thick hydrogel sheet (10 × 20 cm) was washed with 1.5 L of ion-exchanged water in total. The cured 0.5-mm-thick hydrogel sheet was further dehydrated by immersion in 0.5 L of acetone and dried under reduced pressure, thereby obtaining a dry hydrogel sheet ("MA-PVA117 (2.0)-SA (3.4) gel sheet" below). The introduction rate of succinic acid (SA) into the dry PVA sheet (active group introduction rate) was 3.4 mol%.

### Synthesis Example 1-D: Sheet

44 mL of ion-exchanged water was added to 6 g of PVA117, and the mixture was stirred at 80°C for four hours to dissolve PVA117. After cooling to room temperature, 7.5 mL of a 50% glutaraldehyde (GA) solution was added to the aqueous solution of PVA117 and mixed, and 6 mL of a 1 mol/L aqueous solution of hydrochloric acid was further added, followed by mixing quickly, thereby preparing an uncrosslinked polymer solution. The uncrosslinked polymer solution was poured into the space between two glass plates with a 0.5-mm spacer between them and cured at 65°C for seven hours. The cured 0.5-mm-thick hydrogel sheet (10 × 20 cm) was washed with 1.5 L of ion-exchanged water in total. The cured 0.5-mm-thick hydrogel sheet was further dehydrated by immersion in 0.5 L of acetone and dried under reduced pressure, thereby obtaining a dry hydrogel sheet ("GA-crosslinked PVA117 gel sheet" below).

### Activation of Hydroxy Group

### Synthesis Example 1-i: Introduction of Succinic Acid into Molded Article (Particles)

1.5 g of the dry MA-PVA117 (2.0) gel particles (monomer repeating unit: 33 mmol) prepared in Synthesis Example 1-A were placed in a separable flask equipped with a 0.5-L Dimroth condenser, and 100 mL of dimethylsulfoxide (DMSO) was added and stirred with a mechanical stirrer to swell the particles. After stirring in a water bath at 60°C for one hour, 364 mg (3.6 mmol) of triethylamine and 330 mg (3.3 mmol) of succinic anhydride were added, and the mixture was further stirred at 60°C for five hours. After cooling, the particles were filtered and washed twice with 100 mL of DMSO and twice with 100 mL of ion-exchanged water. The particles were then dehydrated by immersion in 100 mL of acetone three times, and the obtained particles were dried under reduced pressure to obtain succinate-introduced (SA-intorduced) dry hydrogel particles. The introduction rate of succinic acid (active group introduction rate) was 10.9 mol% relative to the repeating unit of MA-PVA117 (2.0) ("SA-introduced-MA-PVA117 (2.0) gel particles" below).

### Synthesis Example 1-ii: Introduction of Succinic Acid into Molded Article (Particles)

Succinic acid-introduced, dry hydrogel particles were obtained in the same manner as in Synthesis Example 1-i, except that dried Nor-PVA117 (1.3) gel particles prepared in Synthesis Example 1-B were used. The introduction rate of succinic acid (active group introduction rate) was 8.7 mol% relative to the repeating unit of Nor-PVA117 (1.3) ("SA-introduced-Nor-PVA117 (1.3) gel particles" below).

### Synthesis Example 1-iii: Introduction of Succinic Acid into Molded Article (Sheet)

A succinic acid-introduced, dry hydrogel sheet was obtained in the same manner as in Synthesis Example 1-i, except that the dried GA-crosslinked PVA117 gel sheet prepared in Synthesis Example 1-D was used. The introduction rate of succinic acid (active group introduction rate) was 9.8 mol% relative to the repeating unit of GA-crosslinked PVA117 ("SA-introduced-GA-crosslinked PVA117 gel sheet" below).

### Synthesis Example 1-iv: Introduction of Carbonyl Imidazolyl Group into Molded Article (Particles)

377 mg (2.3 mmol) of carbonyldiimidazole ("CDI" below) was dissolved in 7 g of dry acetonitrile, and 1.0 g of the dry MA-PVA117 (2.0) gel particles (monomer repeating unit: 23 mmol) prepared in Synthesis Example 1-A were added thereto. A reaction was performed in a centrifuge tube at 40°C for four hours with the centrifuge tube shaken, followed by washing with 50 mL of dry acetone four times. The particles washed with acetone were vacuum-dried overnight at room temperature to obtain carbonyl imidazolyl-formed (CI-introduced) hydrogel particles ("CI-introduced-MA-PVA117 (2.0) gel particles" below). Table 1 shows the introduction rate of carbonyl imidazolyl groups (active group introduction rate).

### Conjugate Formation

### Synthesis Example 1-a

1 g of the dry SA-introduced-MA-PVA117 (2.0) gel particles (the amount of succinic acid introduced: about 1.9 mmol) prepared in Synthesis Example 1-i were swollen in an MES buffer (a pH of 5.6) at room temperature overnight. The swollen gel particles were dispersed in 45 mL of an MES buffer, and 0.78 g (6.8 mmol) of N-hydroxysuccinimide and 0.65 g (3.4 mmol) of 1-(3-dimethylaminopropyl)-3-ethylcarbodiimide hydrochloride were added, while being stirred with a magnetic stirrer, followed by shaking at room temperature for one hour. An operation of washing the gel particles with 30 mL of an MES buffer for 10 minutes was performed three times, and then the gel particles were added to 70 mL of a 1 mg/mL gelatin PBS solution. The gel particles were shaken at room temperature for three hours, and an operation of washing the obtained hydrogel particles with 70 mL of ion-exchanged water (heated to 60°C) for 20 minutes was performed twice, thereby obtaining gelatin-conjugated hydrogel particles ("gelatin-SA-introduced-MA-PVA117 (2.0) gel particles" below). Some of the gelatin-SA-introduced-MA-PVA117 (2.0) gel particles were immersed in excess PBS overnight, and the amount of immobilized gelatin per gel weight (100 mg) was measured according to the bicinchoninic acid (BCA) method (BCA Protein Assay Kit, produced by Takara Bio Inc.). The conjugate density of the bioactive substance was 48.5 µg/100 mg gel particles.

### Synthesis Example 1-b

Collagen-conjugated hydrogel particles were obtained in the same manner as in Synthesis Example 1-a, except that collagen was used instead of gelatin ("collagen-SA-introduced-MA-PVA117 (2.0) gel particles" below). The amount of immobilized collagen in collagen-SA-introduced-MA-PVA117 (2.0) gel particles (the conjugate density of the bioactive substance) was also measured according to the same method as in Synthesis Example 1-a.

### Synthesis Example 1-c

Gelatin-conjugated hydrogel particles were obtained in the same manner as in Synthesis Example 1-a, except that 1 g of the dry SA-introduced-Nor-PVA117 (1.3) gel particles prepared in Synthesis Example 1-ii were used ("gelatin-SA-introduced-Nor-PVA117 (1.3) gel particles" below). The amount of immobilized gelatin of the gelatin-SA-introduced Nor-PVA117 (2.0) gel particles (the conjugate density of the bioactive substance) was also measured according to the same method as in Synthesis Example 1-a.

### Synthesis Example 1-d

A gelatin-conjugated hydrogel sheet was obtained in the same manner as in Synthesis Example 1-a, except that 1 g of the dry MA-PVA117 (2.0)-SA (3.4) gel sheet prepared in Synthesis Example 1-C was used ("gelatin-MA-PVA117 (2.0)-SA (3.4) gel sheet" below). The amount of immobilized gelatin of the gelatin-MA-PVA117 (2.0)-SA (3.4) gel sheet (the conjugate density of the bioactive substance) was also measured according to the same method as in Synthesis Example 1-a.

### Synthesis Example 1-e

A gelatin-conjugated hydrogel sheet was obtained in the same manner as in Synthesis Example 1-a, except that 1 g of the dry SA-introduced-GA-crosslinked PVA117 gel sheet prepared in Synthesis Example 1-iii was used ("gelatin-SA-introduced-GA-crosslinked PVA117 gel sheet" below). The amount of immobilized gelatin of the gelatin-SA-introduced-GA-crosslinked PVA117 gel sheet (the conjugate density of the bioactive substance) was also measured according to the same method as in Synthesis Example 1-a.

### Synthesis Example 1-f

0.6 g of gelatin was dissolved in 100 mL of PBS at 60°C with stirring using a magnetic stirrer. The gelatin solution was cooled to room temperature, and the CI-introduced-MA-PVA117 (2.0) gel particles prepared in Synthesis Example 1-iv were added. The mixture was shaken and stirred at room temperature overnight, and washed with 100 mL of PBS three times to obtain gelatin-conjugated gel particles ("gelatin-urethane-bonded-MA-PVA117 (2.0) gel particles" below). The amount of immobilized gelatin of the gelatin-urethane-bonded-MA-PVA117 (2.0) gel particles (the conjugate density of the bioactive substance) was measured according to the same method as in Synthesis Example 1-a.

### Particle Drying and Gamma Ray Sterilization

### Example 1-1

2 g of the gelatin-SA-introduced-MA-PVA117 (2.0) gel particles (swollen with ion-exchanged water) prepared in Synthesis Example 1-a were spread on a Petri dish and dried naturally in air at room temperature for 2.7 hours. The water content of the obtained dry particles was measured with a heat-dry moisture meter (produced by A&D Company, Limited) and was 72 mass%. The dry gel particles were placed in a 15-mL centrifuge tube, further sealed in an aluminum-metallized bag to prevent evaporation of water, and irradiated with gamma rays (25 kGy) at Koga Isotope Ltd., thereby obtaining a sterilized amide bond-conjugated hydrogel. Table 1 shows the results of measuring the water content.

### Examples 1-2 to 1-4

Water content measurement and gamma irradiation were performed to obtain a sterilized amide bond-conjugated hydrogel in the same manner as in Example 1-1, except that the time for naturally drying 2 g of gelatin-SA-introduced-MA-PVA117 (2.0) gel particles (swollen with ion-exchanged water) was changed to 3.7, 4.5, and 5.5 hours. Table 1 shows the results of measuring the water content.

### Example 1-5

2 g of gelatin-SA-introduced-MA-PVA117 (2.0) gel particles (swollen with ion-exchanged water) were spread on a Petri dish and vacuum-dried overnight at room temperature. Water content measurement and gamma irradiation were performed in the same manner as in Example 1-1, thereby obtaining a sterilized amide bond-conjugated hydrogel. Table 1 shows the results of measuring the water content.

### Example 1-6

2 g of the gelatin-SA-introduced-MA-PVA117 (2.0) gel particles (swollen with ion-exchanged water) prepared in Synthesis Example 1-a were immersed in 25 mL of ethanol three times, and the dehydrated conjugated gel particles were vacuum-dried overnight at room temperature. The water content of the obtained dry particles was determined by ¹H-NMR (deuterated DMSO solvent). The water content was determined from the ratio of the integral of the signal of water (3.3 ppm) to the signal of the vinyl alcohol polymer (water originally contained in the deuterated DMSO solvent was subtracted). Gamma irradiation was performed in the same manner as in Example 1-1, thereby obtaining a sterilized amide bond-conjugated hydrogel. Table 1 shows the results of measuring the water content.

### Example 1-7

2 g of the collagen-SA-introduced-MA-PVA117 (2.0) gel particles (swollen with ion-exchanged water) prepared in Synthesis Example 1-b were dried, measured for water content and irradiated with gamma rays in the same manner as in Example 1-6, thereby obtaining a sterilized amide bond-conjugated hydrogel. Table 1 shows the results of measuring the water content.

### Example 1-8

Drying, water content measurement, and gamma irradiation were performed in the same manner as in Example 1-5, except that the gelatin-SA-introduced-Nor-PVA117 (1.3) gel particles (swollen with ion-exchanged water) prepared in Synthesis Example 1-c were used, thereby obtaining a sterilized amide bond-conjugated hydrogel. Table 1 shows the results of measuring the water content.

### Example 1-9

Drying, water content measurement, and gamma irradiation were performed in the same manner as in Example 1-5, except that the gelatin-MA-PVA117 (2.0)-SA (3.4) gel sheet (swollen with ion-exchanged water) prepared in Synthesis Example 1-d was used, thereby obtaining a sterilized amide bond-conjugated hydrogel. Table 1 shows the results of measuring the water content.

### Example 1-10

Drying, water content measurement, and gamma irradiation were performed in the same manner as in Example 1-5, except that the gelatin-SA-introduced-GA-crosslinked PVA117 gel sheet (swollen with ion-exchanged water) prepared in Synthesis Example 1-e was used, thereby obtaining a sterilized amide bond-conjugated hydrogel. Table 1 shows the results of measuring the water content.

### Example 1-11

Drying, water content measurement, and gamma irradiation were performed in the same manner as in Example 1-6, except that the gelatin-urethane-bonded-MA-PVA117 (2.0) gel particles (swollen with ion-exchanged water) prepared in Synthesis Example 1-f were used, thereby obtaining a sterilized conjugated hydrogel. Table 1 shows the results of measuring the water content.

### Evaluation Method for Conjugated Hydrogels Obtained in Examples and Comparative Examples

### Evaluation of Cell Adhesion: Particles

Poly(2-hydroxyethylmethacrylate) was dissolved in 95% ethanol at a concentration of 30 mg/mL, and 200 µL of this solution was dispensed into each well of a 24-well plate for cell culture (IWAKI) and dried in a clean bench. The sterilized amide bond-conjugated hydrogel (spherical particles) obtained in Example 1-1 was then immersed in phosphate buffered saline (PBS) for one hour to allow the particles to swell. 200 µL of the gel particles were placed in one well of the coated well plate, and 500 µL of DMEM medium supplemented with 10% fetal bovine serum was added to the same well. Thereupon, 1.9 × 10⁵ NIH/3T3 cells (purchased from ATCC), which had been grown in pre-culture, were added and cultured at a CO² concentration of 5% under saturated water vapor pressure at 37°C. After 24 hours of incubation, a photograph of the center of the culture well was taken with an inverted microscope equipped with a camera and image consolidation software. The number of particles to which cells were adhered and extended was counted, and cell adhesion and extension were observed in 132 particles out of 200 gel particles. In this case, the cell adhesion rate (%) was defined as the percentage of the number of particles in which cell adhesion and extension were observed out of 200 particles. The cell adhesion rate was 66% (Table 1).

The sterilized conjugated hydrogels (spherical particles) obtained in Examples 1-2 to 1-8 and 1-11 and Comparative Examples 1-1 and 1-2 were evaluated for cell adhesion in the same manner as in the Evaluation of Cell Adhesion: Particles described above. Table 1 shows the results.

### Evaluation of Cell Adhesion: Sheet

After the sterilized amide bond-conjugated hydrogel (sheet) obtained in Example 1-9 was immersed in PBS for one hour to allow the gel sheet to swell, the gel sheet was punched to cut out a circle with a 15-mm-diameter hollow punch, and the cut-out piece was placed on the bottom of a 24-well plate. Additionally, 500 µL of DMEM medium supplemented with 10% fetal bovine serum was added to the same well. On the same plate, a well containing 500 µL of medium only, without the gel sheet, was also prepared. Subsequently, 1.9 × 10⁵ NIH/3T3 cells grown in pre-culture were added to the wells with and without the gel sheet, and cultured at 5% CO² concentration under saturated water vapor pressure at 37°C. After 24 hours of incubation, the medium in the wells was removed with an aspirator, and 500 µL of fresh medium was added, followed by adding 50 µL of Cell Counting Kit-8, which is a reagent for measuring cell growth/cytotoxicity (CCK-8, produced by Dojindo Laboratories). At the same time, 500 µL of fresh medium and 50 µL of CCK-8 were added to unused wells that contained no gel sheet and no cells, and the well plate was incubated for one hour at a CO² concentration of 5% under saturated water vapor pressure at 37°C. After incubation, 100 µL of the medium was taken from each well containing CCK-8 and transferred to a 96-well plate, and absorbance at 450 nm was measured with a microplate reader (ARVO, PerkinElmer). With the absorbance determined based on the wells containing only cells taken as a cell adhesion rate of 100%, and the absorbance determined based on the wells containing only medium taken as a cell adhesion rate of 0%, the cell adhesion rate was calculated from the absorbance determined based on the wells containing the gel sheet and cells, and the result was 90% (Table 1).

The cell adhesion was evaluated for the sterilized amide bond-conjugated hydrogel obtained in Example 1-10 according to the same method as in Example 1-9, and the cell adhesion rate was 87% (Table 1).

### Table 1

**Table 1**

| | | Vinyl Alcohol Polymer | Crosslinking Method | Activation Method | Active Group Introduction Rate (mol%) | Bioactive Substance or Enzyme | Method for Forming Conjugate with Bioactive Substance or Enzyme | Conjugate Density of Bioactive Substance (ug/100 mg Water-containing Hydrogel) | Shape of Hydrogel-forming Article | Drying Method | Water Content (mass%) | Measurement Method for Water Content | Sterilization Method | Cell Adhesion Rate (%) |
|---|---|---|---|---|---|---|---|---|---|---|---|---|---|---|
| Example | 1-1 | M4-PVA117(2.0) | Polymerization Crosslinking | Succinic Anhydride | 10.9 | Gelatin | EDC Activation | 48.5 | Spherical Particles | Natural Drying | 72 | Heat-dry Moisture Meter | Gamma Ray (25 kGy) | 66 |
| | 1-2 | M4-PVA117(2.0) | Polymerization Crosslinking | Succinic Anhydride | 10.9 | Gelatin | EDC Activation | 48.5 | Spherical Particles | Natural Drying | 53 | Heat-dry Moisture Meter | Gamma Ray (25 kGy) | 80 |
| | 1-3 | M4-PVA117(2.0) | Polymerization Crosslinking | Succinic Anhydride | 10.9 | Gelatin | EDC Activation | 48.5 | Spherical Particles | Natural Drying | 34 | Heat-dry Moisture Meter | Gamma Ray (25 kGy) | 90 |
| | 1-4 | M4-PVA117(2.0) | Polymerization Crosslinking | Succinic Anhydride | 10.9 | Gelatin | EDC Activation | 48.5 | Spherical Particles | Natural Drying | 15 | Heat-dry Moisture Meter | Gamma Ray (25 kGy) | 89 |
| | 1-5 | M4-PVA117(2.0) | Polymerization Crosslinking | Succinic Anhydride | 10.9 | Gelatin | EDC Activation | 48.5 | Spherical Particles | Drying under Reduced Presssure | 7 | Heat-dry Moisture Meter | Gamma Ray (25 kGy) | 90 |
| | 1-6 | M4-PVA117(2.0) | Polymerization Crosslinking | Succinic Anhydride | 10.9 | Gelatin | EDC Activation | 48.5 | Spherical Particles | Ethanol Replacement, Drying under Reduced Presssure | 0.1 or less | NMR | Gamma Ray (25 kGy) | 91 |
| | 1-7 | M4-PVA117(2.0) | Polymerization Crosslinking | Succinic Anhydride | 10.9 | Collagen | EDC Activation | 48.5 | Spherical Particles | Ethanol Replacement, Drying under Reduced Presssure | 0.1 or less | NMR | Gamma Ray (25 kGy) | 90 |
| | 1-8 | Nor-PVA117(1.3) | Polymerization Crosslinking | Succinic Anhydride | 8.7 | Gelatin | EDC Activation | 38.8 | Spherical Particles | Drying under Reduced Presssure | 7 | Heat-dry Moisture Meter | Gamma Ray (25 kGy) | 92 |
| | 1-9 | M4-PVA117(2.0)-SA(3.4) | Polymerization Crosslinking | Succinic Anhydride | 3.4 | Gelatin | EDC Activation | 283 | Sheet | Drying under Reduced Presssure | 8 | Heat-dry Moisture Meter | Gamma Ray (25 kGy) | 90 |
| | 1-10 | PVA117 | GA Crosslinking | Succinic Anhydride | 98 | Gelatin | EDC Activation | 182 | Sheet | Drying under Reduced Presssure | 7 | Heat-dry Moisture Meter | Gamma Ray (25 kGy) | 87 |
| | 1-11 | M4-PVA117(2.0) | Polymerization Crosslinking | CDI | 13.5 | Gelatin | CDI Activation | 8.3 | Spherical Particles | Ethanol Replacement, Drying under Reduced Presssure | 0.1 or less | NMR | Gamma Ray (25 kGy) | 85 |
| Comparative Example | 1-1 | M4-PVA117(2.0) | Polymerization Crosslinking | Succinic Anhydride | 10.9 | Gelatin | EDC Activation | 48.5 | Spherical Particles | None | 98 | Heat-dry Moisture Meter | Gamma Ray (25 kGy) | 47 |
| | 1-2 | M4-PVA117(2.0) | Polymerization Crosslinking | Succinic Anhydride | 10.9 | Gelatin | EDC Activation | 48.5 | Spherical Particles | Natural Drying | 92 | Heat-dry Moisture Meter | Gamma Ray (25 kGy) | 52 |

As is clear from the results of Examples 1-1 to 1-11 and Comparative Examples 1-1 and 1-2, the present invention allows the bioactive substance to maintain its activity even after irradiation with high-energy gamma rays and can facilitate cell adhesion and even cell proliferation.

### Synthesis Examples

### Synthesis of Vinyl Alcohol Polymer Having Ethylenically Unsaturated Group

### Synthesis Example 2-1

40 g (monomer repeating unit: 908 mmol) of PVA117 (starting material PVA) was placed in a separable flask equipped with a 1-L Dimroth condenser. 350 mL of dimethylsulfoxide (DMSO) was added, and stirring was started with a mechanical stirrer. After the temperature was raised to 80°C by a water bath, stirring was continued at 80°C for four hours. After the starting material PVA was visually confirmed to have dissolved, 1.2 g (10.9 mol) of vinyl methacrylate was added with heating and stirring at 80°C and further stirred for three hours at 80°C. After cooling, the reaction solution was poured into 2 L of methanol with stirring. Stirring was ended, and the solution was allowed to stand for one hour. The obtained solid was collected and then further washed by immersion in 1 L of methanol for one hour. This washing operation was performed a total of three times. The collected solid was vacuum-dried overnight at room temperature, thereby obtaining methacryloylated PVA117. The introduction rate of an ethylenically unsaturated group (methacryloyl group) into the methacryloylated PVA117 was 1.2 mol% relative to the repeating unit of the starting material PVA ("MA-PVA117 (1.2)" below).

### Synthesis Examples 2-2 and 2-3

A vinyl alcohol polymer having an ethylenically unsaturated group was produced in the same manner as in Synthesis Example 2-1, except that the amount of vinyl methacrylate added was changed, as shown in Table 2.

### Synthesis Example 2-4

A vinyl alcohol polymer having an ethylenically unsaturated group was produced in the same manner as in Synthesis Example 2-1, except that starting material PVA (AF-17), in which itaconic acid was copolymerized to incorporate a carboxy group, was used, as shown in Table 2.

### Synthesis Example 2-5

10 g (monomer repeating unit: 220 mmol) of the MA-PVA117 (2.0) prepared in Synthesis Example 2-2 was placed in a separable flask equipped with a 0.5-L Dimroth condenser. 90 mL of dimethylsulfoxide (DMSO) was added, and stirring was started with a mechanical stirrer. After the temperature was raised to 80°C by a water bath, stirring was continued at 80°C for four hours. After the starting material PVA was visually confirmed to have dissolved, the water bath was set to 60°C. After the internal temperature was confirmed to have reached 60°C, 1.2 g (12.1 mmol) of triethylamine and 1.1 g (11 mmol) of succinic anhydride were added and further stirred at 60°C for five hours. After cooling, the reaction solution was poured into 0.5 L of methanol with stirring. Stirring was ended, and the solution was allowed to stand for one hour. The obtained solid was collected and further washed by immersion in 0.5 L of methanol for one hour. This washing operation was performed a total of three times. The collected solid was vacuum-dried overnight at room temperature, thereby obtaining succinic acid-introduced methacryloylated PVA117. The introduction rate of succinic acid (SA) was 3.4 mol% relative to the repeating unit of MA-PVA117 (2.0) ("MA-PVA117 (2.0)-SA (3.4)" below).

### Synthesis Example 2-6

60 g (monomer repeating unit: 1.36 mol) of PVA117 (starting material PVA) was placed in a separable flask equipped with a 1-L Dimroth condenser. 540 mL of ion-exchanged water was added, and stirring was started with a mechanical stirrer. After the temperature was raised to 80°C by a water bath, stirring was continued at 80°C for four hours. After the starting material PVA was visually confirmed to have dissolved, the temperature was lowered to 40°C. With stirring at 40°C, 2.5 g (20.5 mmol) of 5-norbornene-2-carboxyaldehyde and 22 mL of a 10 vol% aqueous solution of sulfuric acid were added, and the mixture was further stirred at 40°C for four hours. After cooling, the solution was neutralized by adding 80 mL of a 1N aqueous solution of NaOH and desalted in a dialysis membrane with a fractional molecular weight of 3,500 (performed four times for 5 L of ion-exchanged water). The desalted aqueous solution was poured into 2 L of methanol with stirring and allowed to stand for one hour. The obtained solid was collected and further washed by immersion in 1 L of methanol for one hour. The collected solid was vacuum-dried overnight at room temperature, thereby obtaining a norbornene-introduced PVA117. The introduction rate of an ethylenically unsaturated group (norbornenyl group (Nor)) into the norbornene-introduced PVA117 was 1.3 mol% relative to the repeating unit in the starting material PVA ("Nor-PVA117 (1.3)" below).

### Production of Molded Article: Particles, Sheets, Coating, and Tubes

### Synthesis Example 2-A: Particles

440 mL of ion-exchanged water was added to 60 g of MA-PVA117 (1.2), and the mixture was stirred at 80°C for four hours to dissolve MA-PVA117 (1.2). After cooling to room temperature, potassium persulfate, which is a water-soluble thermal radical polymerization initiator, was added to give 0.1 mass% to the aqueous solution of MA-PVA117 (2.0), thereby preparing an uncrosslinked gel solution.

3300 mL of liquid paraffin and 8 g of Span 80 were placed in a separable flask with a 5-L Dimroth condenser, and then the uncrosslinked gel solution was slowly added thereto. The mixture was stirred at 350 rpm with a mechanical stirrer to form a W/O dispersion. After the temperature was raised to 40°C by a water bath, purging with nitrogen was performed for 30 minutes. Thereafter, stirring was continued at 70°C for three hours. After cooling to room temperature, the liquid paraffin in which hydrogel particles were dispersed was filtered through a mesh with an opening of 100 um. The obtained hydrogel particles were washed with 3 L of hexane in total to remove the liquid paraffin. The obtained hydrogel particles were then classified into particle sizes of 180 to 300 um by using JIS standard sieves. The hydrogel particles were further added to 1 L of acetone, dehydrated, and then dried under reduced pressure at room temperature overnight, thereby obtaining dry hydrogel particles ("MA-PVA117 (1.2) gel particles" below).

### Synthesis Examples 2-B to 2-E: Particles

Methacryloylated PVA was produced in the same manner as in Synthesis Example 2-1, except that the vinyl alcohol polymers having an ethylenically unsaturated group synthesized in Synthesis Examples 2-2 to 2-5 were used, as shown in Table 3.

### Synthesis Example 2-F: Particles

In Synthesis Example 2-A, 440 mL of ion-exchanged water was added to 60 g of Nor-PVA117 (1.3), and the mixture was stirred at 80°C for four hours to dissolve Nor-PVA117 (1.3). After cooling to room temperature, 3.4 g of 3,6-dioxa-1,8-octanedithiol (polythiol) was added to the aqueous solution of Nor-PVA117 (1.3) and stirred. Potassium persulfate, which is a water-soluble thermal radical polymerization initiator, was added to this solution to give 0.1 mass% and dissolved to prepare an uncrosslinked gel solution. Dry hydrogel particles were obtained by using the uncrosslinked gel solution according to the same method as in Synthesis Example 2-A ("Nor-PVA117 (1.3) gel particles" below).

### Synthesis Example 2-G: Sheet

44 mL of ion-exchanged water was added to 6 g of MA-PVA117 (2.0)-SA (3.4), and the mixture was stirred at 80°C for four hours to dissolve MA-PVA117 (2.0)-SA (3.4). After cooling to room temperature, potassium persulfate, which is a water-soluble thermal radical polymerization initiator, was added to the aqueous solution of MA-PVA117 (2.0) to give 0.1 mass% and dissolved to prepare an uncrosslinked gel solution. The uncrosslinked gel solution was poured into the space between two glass plates with a 0.5-mm spacer between them in a nitrogen atmosphere and cured at 40°C for 3 hours. The cured hydrogel sheet (10 × 20 cm) was washed with 300 mL of ion-exchanged water five times. The cured 0.5-mm-thick hydrogel sheet was further dehydrated by immersion in 1 L of acetone and dried under reduced pressure at room temperature overnight, thereby obtaining a dry hydrogel sheet ("MA-PVA117 (2.0)-SA (3.4) gel sheet" below).

### Synthesis Example 2-H: Coating

100 mL of ion-exchanged water was added to 0.15 g of MA-PVA117 (2.0)-SA (3.4), and the mixture was stirred at 80°C for four hours to dissolve MA-PVA117 (2.0)-SA (3.4). The solution was cooled to room temperature to prepare an uncrosslinked gel solution. The uncrosslinked gel solution was added at 1 mL/well to a polystyrene 6-well plate for cell culture (adhesive cell culture plate produced by Sumitomo Bakelite Co., Ltd.) and dried at room temperature for three days. A 0.1 wt% solution of Omnirad 2959 in methanol was added at 1 mL/well and irradiated with UV light (103 mW/cm², 520 mJ/cm² in UV intensity measurement with a UVR-T1 (UD-T36) metal halide lamp, produced by GS Yuasa International Ltd.). The methanol solution was removed, and washing was performed with 2 mL of ion-exchanged water twice to obtain a 6-well plate coated with the hydrogel ("MA-PVA117 (2.0)-SA (3.4) gel coating" below).

### Activation of Hydroxy Groups

### Synthesis Example 2-i: Introduction of Succinic Acid into Molded Article (Particles)

1.5 g of the dry MA-PVA117 (1.2) gel particles (monomer repeating unit: 33 mmol) prepared in Synthesis Example 2-A were placed in a separable flask equipped with a 0.5-L Dimroth condenser. 100 mL of dimethylsulfoxide (DMSO) was added and stirred with a mechanical stirrer to allow the dry MA-PVA117 (1.2) gel particles to swell. After stirring at 60°C in a water bath for one hour, 364 mg (3.6 mmol) of triethylamine and 330 mg (3.3 mmol) of succinic anhydride were added, and the mixture was further stirred at 60°C for five hours. After cooling, the particles were filtered off and washed with 100 mL of DMSO twice and with 100 mL of water twice. Subsequently, the particles were dehydrated by immersion in 100 mL of acetone three times, and the obtained particles were dried under reduced pressure, thereby obtaining succinic acid-introduced dry hydrogel particles. The introduction rate of the carboxy group (succinic acid) (the introduction density of carboxy groups) was 9.9 mol% relative to the repeating unit of MA-PVA117 (1.2) ("SA-introduced-MA-PVA117 (1.2) gel particles" below).

### Synthesis Examples 2-ii to 2-iv: Introduction of Succinic Acid into Molded Article (Particles)

Succinic-acid-introduced dry hydrogel particles were obtained in the same manner as in Synthesis Example 2-i, except that the gel particles prepared in Synthesis Examples 2-B, 2-C, and 2-F were used as shown in Table 4. Table 4 also shows the results of the introduction rate of the carboxy group (succinic acid) (the introduction density of the carboxy group).

### Conjugate Formation

### Example 2-1

1 g of the dry SA-introduced-MA-PVA117 (1.2) gel particles (succinic acid introduced: about 1.9 mmol) prepared in Synthesis Example 2-i were allowed to swell in MES buffer (a pH of 5.6) at room temperature overnight. While the swollen gel particles were dispersed in 45 mL of MES buffer and stirred with the magnetic stirrer, 0.78 g (6.8 mmol) of N-hydroxysuccinimide and 0.65 g (3.4 mmol) of 1-(3-dimethylaminopropyl)-3-ethylcarbodiimide hydrochloride were added, and the mixture was shaken at room temperature for one hour. An operation of washing the gel particles with 30 mL of MES buffer for 10 minutes was performed three times, and the gel particles were added to 70 mL of a 1 mg/mL gelatin PBS solution. An operation of shaking the mixture at room temperature for 3 hours and washing the obtained hydrogel particles with 70 mL of ion-exchanged water (heated to 60°C) for 20 minutes was performed twice. The hydrogel particles were further immersed in 50 mL of ethanol three times and then vacuum-dried at room temperature overnight, thereby obtaining dry amide-bound gelatin-conjugated hydrogel particles ("gelatin-SA-introduced-MA-PVA117 (1.2) gel particles" below). Some of the gelatin-SA-introduced-MA-PVA117 (1.2) gel particles were immersed in excess PBS overnight, and the amount of immobilized gelatin per gel weight (100 mg) (the conjugate density of the bioactive substance) was measured according to the bicinchoninic acid (BCA) method (BCA Protein Assay Kit, produced by Takara Bio Inc.). The conjugate density of the bioactive substance was 48.2 µg/100 mg gel particles.

### Examples 2-2 to 2-6

Amide-bound gelatin-conjugated hydrogel particles were obtained in the same manner as in Example 2-1, except that the carboxy group-introduced hydrogel particles of Synthesis Examples 2-ii to 2-iv, 2-D, and 2-E were used. Table 5 shows the results of measuring the amount of immobilized gelatin (the conjugate density of the bioactive substance) according to the BCA method.

### Example 2-7

Collagen-conjugated PVA particles were obtained in the same manner as in Example 2-1, except that collagen was used instead of gelatin ("collagen-SA-introduced-MA-PVA117 (1.2) gel particles" below). The amount of immobilized collagen (the conjugate density of the bioactive substance) was measured according to the BCA method. The conjugate density of the bioactive substance was 53.2 µg/100 mg gel particles.

### Example 2-8: Gelatin-conjugated Sheet

An amide-bound gelatin-conjugated hydrogel sheet was obtained in the same manner as in Example 2-1, except that 1 g of the dry MA-PVA117 (2.0)-SA (3.4) gel sheet prepared in Synthesis Example 2-G was used ("gelatinized-MA-PVA117 (2.0)-SA (3.4) gel sheet" below). The amount of immobilized gelatin (the conjugate density of the bioactive substance) was measured according to the BCA method. The conjugate density of the bioactive substance was 20.0 µg/100 mg gel particles.

### Example 2-9: Gelatin-conjugated Coating

251 mg (2.2 mmol) of N-hydroxysuccinimide was dissolved in 25 mL of MES buffer, and then 425 mg (2.2 mmol) of 1-(3-dimethylaminopropyl-3-ethylcarbodiimide hydrochloride was added and stirred to dissolve it, thereby preparing a condensation agent solution. This condensation agent solution was added at 1 mL/well to the MA-PVA117 (2.0)-SA (3.4) gel coating prepared in Synthesis Example 2-H, followed by shaking at room temperature for one hour. An operation of washing with 1 mL of MES buffer for 5 minutes was performed twice, and the MES buffer was removed from the wells. A 1 mg/mL gelatin PBS solution was added at 1 mL/well, and shaking was performed at room temperature for three hours. Thereafter, an operation of washing the wells with 2 mL of ion-exchanged water (heated to 60°C) for 5 minutes was performed twice, thereby obtaining an amide-bound gelatin-conjugated hydrogel coating ("gelatin-SA-introduced-MA-PVA117 (2.0) gel coating" below). The amount of immobilized gelatin (the conjugate density of the bioactive substance) was measured according to the BCA method and was found to be 16.6 pg/well.

### Comparative Examples

### Comparative Synthesis Example 2-1

1.0 g of the dry MA-PVA117 (2.0) gel particles (monomer repeating unit: 23 mmol) prepared in Synthesis Example 2-B were added to 45 mL of ion-exchanged water and shaken in a centrifuge tube to allow the gel particles to swell. 30 µL of aminoacetaldehyde dimethylacetal (0.28 mmol) and 2 L of a 17 mass% aqueous solution of sulfuric acid were added thereto, and the mixture was shaken and stirred at 40°C for 10 hours. The particles were filtered off and washed with 100 mL of dry acetone five times. The particles were then washed with 50 mL of acetone four times and vacuum-dried overnight at room temperature, thereby obtaining aminated hydrogel particles. The introduction rate of the amino group (the introduction density of the amino group) was 7.3 mol% relative to the repeating unit of MA-PVA117 (1.2) ("aminated-MA-PVA117 (2.0) gel particles" below).

### Comparative Synthesis Example 2-2: GA-crosslinked Particles

Production was performed with reference to the Journal of Applied Science, 2013, Vol. 13, pp. 2676-2681. 3300 mL of liquid paraffin and 8 g of Span 80 were added to a separable flask equipped with a 5-L Dimroth condenser and stirred at 200 rpm with a mechanical stirrer. Subsequently, 440 mL of ion-exchanged water was added to 60 g of AF-17, and AF-17 was dissolved with stirring at 80°C for four hours. After cooling to room temperature, 75 g of a 50% aqueous solution of glutaraldehyde ("GA" below) and 60 mL of a 1 mol/L aqueous solution of hydrochloric acid were added thereto, and the mixture was immediately added to liquid paraffin. A W/O dispersion was formed and heated to 65°C in a water bath and stirred for seven hours. After cooling to room temperature, the liquid paraffin in which hydrogel particles were dispersed was filtered through a mesh with an opening of 100 um. The obtained hydrogel particles were washed with 3 L of hexane in total to remove the liquid paraffin. The obtained hydrogel particles were then classified into particle sizes of 180 to 300 um by using JIS standard sieves. The hydrogel particles were further added to 1 L of acetone, dehydrated, and then dried under reduced pressure at room temperature overnight, thereby obtaining dry hydrogel particles ("GA-crosslinked AF-17 gel particle" below).

### Comparative Synthesis Example 2-3: Introduction of Carbonyl Imidazolyl Groups into Molded Article (Particles)

377 mg (2.3 mmol) of carbonyldiimidazole ("CDI" below) was dissolved in 7 g of dry acetonitrile, and 1.0 g of the dry MA-PVA117 (2.0) gel particles (monomer repeating unit: 23 mmol) prepared in Synthesis Example 2-B were added thereto. The mixture was allowed to react in a centrifuge tube with shaking at 40°C for four hours and then washed with 50 mL of dry acetone four times. The particles washed with acetone were vacuum-dried at room temperature overnight, thereby obtain carbonyl imidazolyl-introduced (CI-introduced) hydrogel particles ("CI-introduced-MA-PVA117 (2.0) gel particles" below).

### Comparative Example 2-1

1 g of the dry aminated-MA-PVA117 (2.0) gel particles prepared in Comparative Synthesis Example 2-1 were allowed to swell in 45 mL of PBS buffer (a pH of 7.4) at room temperature overnight. Subsequently, while 70 mL of a 1 mg/mL gelatin MES buffer solution was stirred with a magnetic stirrer, 0.78 g (6.8 mmol) of N-hydroxysuccinimide and 0.65 g (3.4 mmol) of 1-(3-dimethylaminopropyl)-3-ethylcarbodiimide hydrochloride were added thereto, and the mixture was shaken at room temperature for one hour to prepare an activated gelatin solution. The aminated-MA-PVA117 (2.0) gel particles swollen in PBS buffer were filtered off, and the gel particles were added to the activated gelatin solution, followed by stirring with a magnetic stirrer at room temperature for 3 hours. An operation of washing the obtained hydrogel particles with 70 mL of ion-exchanged water (heated to 60°C) for 20 minutes was performed twice to obtain amide-bound gelatin-conjugated hydrogel particles ("gelatin-aminated-MA-PVA117 (2.0) gel particles" below). Some of the gelatin-aminated-MA-PVA117 (2.0) gel particles were immersed in excess PBS overnight, and the amount of immobilized gelatin (the conjugate density of the bioactive substance) was measured according to the BCA method. The conjugate density of the bioactive substance was 5.1 µg/100 mg gel particles.

### Comparative Example 2-2

Amide-bound gelatin-conjugated hydrogel particles were obtained in the same manner as in Example 2-1, except that GA-crosslinked AF-17 gel particles were used instead of SA-introduced-MA-PVA117 (1.2) gel particles ("gelatin-GA-crosslinked PVA117 gel particles" below). The amount of immobilized gelatin (the conjugate density of the bioactive substance) was measured according to the BCA method. The conjugate density of the bioactive substance was 1.5 µg/100 mg gel particles.

### Comparative Example 2-3

0.6 g of gelatin was dissolved in 100 mL of PBS at 60°C with stirring with a magnetic stirrer. The gelatin solution was cooled to room temperature, and the CI-introduced-MA-PVA117 (2.0) gel particles prepared in Comparative Synthesis Example 2-3 were added thereto, followed by shaking and stirring the mixture at room temperature overnight. The mixture was then washed with 100 mL of PBS three times, thereby obtaining gelatin-conjugated gel particles ("gelatin-CI-introduced-MA-PVA117 (2.0) gel particles" below). The amount of immobilized gelatin (the conjugate density of the bioactive substance) was measured in the same manner as in Example 2-1. The conjugate density of the bioactive substance was 7.2 µg/100 mg gel particles.

### Method for Evaluating Amide-bound Conjugated Hydrogel Obtained in Examples and Comparative Examples

### Evaluation of Cell Adhesion (Cell Growth Rate)

30 mg of the dry SA-introduced-MA-PVA117 (1.2) gel particles obtained in Example 2-1 were immersed in 5 mL of PBS overnight. The degree of swelling of the particles (weight when swollen/weight when dry) and the average particle size when swollen were measured, and the total surface area of the swollen particle per gram of the dry particle was calculated. Subsequently, 30 mL of DMEM medium supplemented with 10% fetal bovine serum was added to a 125-mL spinner flask (Corning Incorporated), and the dry SA-introduced-MA-PVA117 (1.2) gel particles prepared in Example 2-1 were weighed and added thereto such that the total surface area was 162 cm² based on the total surface area calculated beforehand. Additionally, 8.1 × 10⁵ NIH/3T3 cells (purchased from ATCC), which had been grown in pre-culture, were added and cultured in an incubator at a CO² concentration of 5% under saturated water vapor pressure at 37°C with stirring at a paddle speed of 60 rpm. On the fourth day of culture, all of the gel particles were collected, washed with PBS, and the cells were detached from the gel particles by trypsin treatment. The cell density of the obtained cell suspension was counted with a hemocytometer, and the number of cells after culture was calculated. The cell growth rate was defined as the number of cells after culture/the number of initial cells, and the cell growth rate for the gel particles obtained in Example 2-1 was 10.6. Table 5 shows the results of measuring the cell growth rate of the gel particles of Examples 2-2 to 2-7 and Comparative Examples 2-1 to 2-3 in the same manner.

The cell growth rate of the gelatinized-MA-PVA117 (2.0)-SA (3.4) gel sheet obtained in Example 2-8 was measured according to the following method. After the gel sheet was immersed in PBS overnight and allowed to swell, the gel sheet was punched to cut out a circle with a 34-mm-diameter hollow punch, and the cut-out piece was placed on the bottom of a polystyrene 6-well plate for cell culture. A medium was added to the wells at 3 mL/well, and 4.9 × 10⁶ NIH/3T3 cells grown in pre-culture were added and cultured in an incubator at 5% CO² concentration under saturated water vapor pressure at 37°C. On the fourth day of culture, the cells were detached and collected from the surface of the gel sheet by trypsin treatment. The number of cells after culture was counted, and the cell growth rate calculated was 4.3.

The cell growth rate of the gelatin-SA-introduced MA-PVA117 (2.0) gel coating obtained in Example 2-9 was measured according to the following method. A medium was added at 3 mL/well to a gel-coated polystyrene 6-well plate for cell culture, and 4.9 × 10⁶ NIH/3T3 cells grown in pre-culture were added and cultured in an incubator at 5% CO² concentration under saturated water vapor pressure at 37°C. On the fourth day of culture, the cells were detached and collected from the well plate by trypsin treatment. The number of cells after culture was counted, and the cell growth rate calculated was 5.8.

As is clear from the results of Examples 2-1 to 2-9, and Comparative Examples 2-1 to 2-3, the present invention allows the bioactive polypeptide to maintain its activity even after irradiation with high-energy gamma rays and can facilitate cell proliferation.

**Table 2**

| | Vinyl Alcohol Polymer Having Ethylenically Unsaturated Group | | | |
|---|---|---|---|---|
| | Type | Starting Material PVA | Introduction Rate of Ethylenically Unsaturated Group (mol%) | Introduction Rate of Carboxy Group (mol%) |
| Synthesis Example 2-1 | MA-PVA117(1.2) | PVA117 | 1.2 | - |
| Synthesis Example 2-2 | MA-PVA117(2.0) | PVA117 | 2.0 | - |
| Synthesis Example 2-3 | MA-PVA117(3.0) | PVA117 | 2.9 | - |
| Synthesis Example 2-4 | MA-AF-17(2.0) | AF-17 | 2.1 | 2.6 *Note 1 |
| Synthesis Example 2-5 | MA-PVA117(2.0)-SA(3.4) | PVA117 | 2.0 | 3.4 |
| Synthesis Example 2-6 | Nor-PVA117(1.3) | PVA117 | 1.3 | - |

| | | | | |
|---|---|---|---|---|
| Note 1: Scientific Reports, 2017, Vol. 7, p. 45146 | | | | |

**Table 3**

| | Article | |
|---|---|---|
| | Contracted Name | Vinyl Alcohol Polymer Having Ethylenically Unsaturated Group |
| Synthesis Example 2-A | MA-PVA117(1.2) Gel Particle | MA-PVA117(1.2) |
| Synthesis Example 2-8 | MA-PVA117(2.0) Gel Particle | MA-PVA117(2.0) |
| Synthesis Example 2-C | MA-PVA117(3.0) Gel Particle | MA-PVA117(3.0) |
| Synthesis Example 2-D | MA-AF-17(2.0) Gel Particle | MA-AF-17(2.0) |
| Synthesis Example 2-E | MA-PVA117(2.0)-SA(3.4) Gel Particle | MA-PVA117(2.0)-SA(3.4) |
| Synthesis Example 2-F | Nor-PVA117(1.3) Gel Particle | Nor-PVA117(1.3) |
| Synthesis Example 2-G | MA-PVA117(2.0)-SA(3.4) Gel Sheet | MA-PVA117(2.0)-SA(3.4) |
| Synthesis Example 2-H | MA-PVA117(2.0)-SA(3.4) Gel Coating | MA-PVA117(2.0)-SA(3.4) |

**Table 4**

| | Article Having Carboxy Group Introduced (Particle) | |
|---|---|---|
| | Contracted Name of Particle | Introduction Rate of Carboxy Group (Succinic Acid) (mol%) |
| Synthesis Example 2-i | SA-introduced-MA-PVA117(1.2) Gel Particle | 9.9 |
| Synthesis Example 2-ii | SA-introduced-MA-PVA117(2.0) Gel Particle | 10.3 |
| Synthesis Example 2-iii | SA-introduced-MA-PVA117(3.0) Gel Particle | 7.8 |
| Synthesis Example 2-iv | SA-introduced-Nor-PVA117(1.3) Gel Particle | 9.5 |

### Table 5

**Table 5**

| | | Vinyl Alcohol Polymer | Crosslinking Method | Method for Introducing Carboxy Group | Carboxy-containing PVA Gel Article | Introduction Density of Carboxy Group (mol %, relative to all structural units) | Introduction Density of Amino Group (mol %, relative to all structural units) | Bioactive Substance Having Amino Group | Method for Forming Conjugate with Bioactive Substance or Enzyme | Shape of Hydrogel - forming Article | Conjugate Density of Bioactive Substance (ug/100 mg water-containing hydrogel) | Cell Growth Rate (the number of cells after culture/the number of initial cells) |
|---|---|---|---|---|---|---|---|---|---|---|---|---|
| Example | 2-1 | MA-PVA117(1.2) | Polymerization Crosslinking | Succinic Anhydride Post-modification | SA-introduced-MA-PVA117(1.2) Gel Particle | 9.9 | - | Gelatin | EDC Activation | Spherical Particles | 48.2 | 10.6 |
| | 2-2 | M4-PVA117(2.0) | Polymerization Crosslinking | Succinic Anhydride Post-modification | SA-introduced-MA-PVA117(2.0) Gel Particle | 103 | - | Gelatin | EDC Activation | Spherical Particles | 56.2 | 3.8 |
| | 2-3 | MA-PVA117(3.0) | Polymerization Crosslinking | Succinic Anhydride Post-modification | SA-introduced-MA-PVA117(3.0) Gel Particle | 7.8 | - | Gelatin | EDC Activation | Spherical Particles | 30.7 | 52 |
| | 2-4 | Nor-PVA117(1.3) | Polymerization Crosslinking | Succinic Anhydride Post-modification | SA-introduced-Nor-PVA117(1.3) Gel Particle | 9.5 | - | Gelatin | EDC Activation | Spherical Particles | 45.3 | 9.3 |
| | 2-5 | MA-AF-17(2.0) | Polymerization Crosslinking | Copolymerization Modification | MA-AF-17(2.0) Gel Particle | 2.6 | - | Gelatin | EDC Activation | Spherical Particles | 8 | 4 |
| | 2-6 | M4-PVA117(2.0)-SA(3.4) | Polymerization Crosslinking | Succinic Anhydride Pre-modification | M4-PVA117(2.0)-SA(3.4) Gel Particle | 3.4 | - | Gelatin | EDC Activation | Spherical Particles | 40.5 | 5.8 |
| | 2-7 | MA-PVA117(1.2) | Polymerization Crosslinking | Succinic Anhydride Pre-modification | SA-introduced-MA-PVA117(1.2) Gel Particle | 9.9 | - | Collagen | EDC Activation | Spherical Particles | 53.2 | 8.3 |
| | 2-8 | M4-PVA117(2.0)-SA(3.4) | Polymerization Crosslinking | Succinic Anhydride Pre-modification | M4-PVA117(2.0)-SA(3.4) Gel Sheet | 3.4 | - | Gelatin | EDC Activation | Sheet | 20 | 4.3 |
| | 2-9 | M4-PVA117(2.0)-SA(3.4) | Polymerization Crosslinking | Succinic Anhydride Pre-modification | M4-PVA117(2.0)-SA(3.4) Gel Coating | 3.4 | - | Gelatin | EDC Activation | Coating | 16.6 *Note 1 | 5.8 |
| Comparative Example | 2-1 | M4-PVA117(2.0) | Polymerization Crosslinking | Amino Group Post-modification | Aminated-MA-PVA117(2.0) Gel Particle | - | 7.3 | Gelatin | EDC Activation | Spherical Particles | 5.1 | 0.9 |
| | 2-2 | AF-17 | Glutaraldehyde | Copolymerization Modification | GA-crosslinked AF-17 Gel Particle | 2.6 | - | Gelatin | EDC Activation | Spherical Particles | 1.5 | 1.2 |
| | 2-3 | M4-PVA117(2.0) | Polymerization Crosslinking | - | CI-introduced-MA-PVA117(2.0) Gel Particle | - | - | Gelatin | CDI Activation | Spherical Particles | 7.2 | 0.8 |

Note 1: Bioactive substance contained in a single well (ug)

### Sterilization Validation (VDₘₐₓ Method)

### Example 3

Gelatin-conjugated hydrogel particles were obtained in the same manner as in Synthesis Example 1-a, except that synthesis was performed in a class 10000 clean room, all buffers and ion-exchanged water underwent filtration sterilization using a membrane filter with a pore size of 0.2 um, and 30 g of the dry SA-introduced-MA-PVA117 (2.0) gel particles prepared in Synthesis Example 1-i were used ("gelatin-SA-introduced-MA-PVA117 (2.0) gel particles" below). The amount of immobilized gelatin per gel weight (100 mg) was measured according to the bicinchoninic acid (BCA) method, and a conjugate density of the bioactive substance was 56.3 µg/100 mg gel particles. The obtained swollen gel particles were dried in the same manner as in Example 1-6. The water content, measured in the same manner as in Example 1-6, was 0.1 wt%.

The dry hydrogel particles obtained above were sterilized according to the VDₘₐₓ²⁵ method. Specifically, 1 g of the dry hydrogel particles were placed in a sterilized 25-mL centrifuge tube and covered with a lid. In this manner, 25 tubes of dry hydrogel particles kept in a container were prepared. The average bioburden of 10 tubes among these was measured and found to be 7.6. A sterility test was performed by irradiating 10 tubes with a gamma dose of 6.9 kGy in VDₘₐₓ²⁵, which corresponds to an average bioburden of 8.0, and 1 out of 10 tubes was positive (substantiation of SAL = 10⁻¹). Because the sterilization dose was found to be 25 kGy from this result, five tubes containing the dry hydrogel particles were irradiated with gamma rays of 25 kGy or higher, thereby obtaining sterilized dry hydrogel particles that satisfied SAL = 10⁻⁶.

### Industrial Applicability

In the first embodiment, because the dry hydrogel-forming article of the present invention is sterilized, the dry hydrogel-forming article has no risk of microbial infection, and can facilitate cell adhesion and can further allow for efficient cell proliferation or induction into tissues or organs. Thus, the dry hydrogel-forming article of the present invention is useful as a carrier or support. In the second embodiment, because the hydrogel of the present invention is efficiently conjugated with a bioactive substance, the hydrogel allows for efficient cell proliferation or induction into tissues or organs. Thus, the hydrogel of the present invention is useful as a carrier or support. Accordingly, carriers containing the dry hydrogel-forming article of the present invention and carriers containing the hydrogel of the present invention are suitable for use in enzyme immobilization carriers, affinity carriers, cell culture carriers, drug delivery carriers, etc.

## Claims

1. A sterilized dry hydrogel-forming article comprising a crosslinked product of a vinyl alcohol polymer having a bioactive substance conjugated thereto.

2. The dry hydrogel-forming article according to claim 1, wherein the bioactive substance is conjugated to the crosslinked product of the vinyl alcohol polymer via a covalent bond.

3. The dry hydrogel-forming article according to claim 1 or 2, wherein the dry hydrogel-forming article has a water content of 75 mass% or less.

4. The dry hydrogel-forming article according to any one of claims 1 to 3, wherein the dry hydrogel-forming article is non-spherical particles, spherical particles, a fine molded article, an article of any shape molded with a 3D printer, a film, thread, hollow fibers, a porous monolith, or a coated article.

5. The dry hydrogel-forming article according to any one of claims 1 to 4, wherein the dry hydrogel-forming article has a sterility assurance level (SAL) of 10⁻³ or less.

6. A method for producing the dry hydrogel-forming article of any one of claims 1 to 5, the method comprising sterilizing a hydrogel-forming article in a dry state.

7. The method according to claim 6, wherein the sterilization is performed by a radiation sterilization method.

8. The method according to claim 7, wherein the radiation dose is 8.2 kilo Grays (kGy) of or more.

9. The method according to claim 7 or 8, wherein the dry hydrogel-forming article is kept in a container and then irradiated with radiation.

10. The dry hydrogel-forming article according to any one of claims 1 to 5, which is kept in a container.

11. A hydrogel comprising
a crosslinked product of a vinyl alcohol polymer having an ethylenically unsaturated group and a carboxy group and
a bioactive substance having an amino group,
the carboxy group of the crosslinked product and the amino group of the bioactive substance being covalently bound via an amide bond.

12. The hydrogel according to claim 11, wherein the ethylenically unsaturated group is at least one member selected from the group consisting of vinyl, (meth)acryloyl, (meth)acryloylamino, vinylphenyl, norbornenyl, and derivatives thereof.

13. The hydrogel according to claim 11 or 12, wherein an introduction rate of the ethylenically unsaturated group is 0.01 to 10 mol%, based on all structural units constituting the vinyl alcohol polymer.

14. The hydrogel according to any one of claims 11 to 13, wherein an introduction rate of the carboxy group is 0.1 to 50 mol%, based on all structural units constituting the vinyl alcohol polymer.

15. The hydrogel according to any one of claims 11 to 14, wherein the hydrogel is non-spherical particles, spherical particles, a fine molded article, an article of any shape molded with a 3D printer, a film, thread, hollow fibers, a porous monolith, or a coated article.
